# EUROPEAN PATENT APPLICATION

(11) **EP 4 506 362 A1**
(43) Date of publication of application: **12.02.2025**
(21) Application number: 23778443.4
(22) Date of filing: 31.03.2023
(51) Int. Cl.: C07K 16/28, C07K 19/00, C12N 15/85, C12N 15/62, C12N 5/10, A61K 38/17, A61K 39/395, A61P 35/00

(54) **CD80 PROTEIN VARIANT AND CD80 FUSION PROTEIN**

(30) Priority: 02.04.2022 CN 202210355272
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: LV, Ming, Hangzhou, Zhejiang 310056 (CN); DING, Xiaoran, Hangzhou, Zhejiang 310056 (CN); MIAO, Shiwei, Hangzhou, Zhejiang 310056 (CN); TAN, Bin, Hangzhou, Zhejiang 310056 (CN); TAO, Jun, Hangzhou, Zhejiang 310056 (CN); CUI, Hang, Hangzhou, Zhejiang 310056 (CN)
(74) Representative: Weickmann & Weickmann PartmbB
(86) International application number: PCT/CN2023/085423
(87) International publication number: WO 2023/186079

(57) **Abstract**

Provided are a CD80 protein variant and a fusion protein comprising the CD80 protein variant, which can bind to PD-L1, CTLA-4 and CD28, activate the activity of T lymphocytes, and inhibit the growth and/or proliferation of tumors or tumor cells. Also provided are a nucleic acid encoding the fusion protein, a vector comprising the fusion protein, a cell comprising the nucleic acid or the vector, a method for preparing the fusion protein, and use of the fusion protein.

## Description

### TECHNICAL FIELD

The present application relates to the field of biopharmaceuticals, and particularly to a CD80 protein variant and a fusion protein comprising the CD80 variant.

### BACKGROUND

CD80, also known as B7, B7.1, or BB1, belongs to the immunoglobulin superfamily, and is mainly expressed in activated B lymphocytes, activated T lymphocytes, macrophages, and the like. The receptors for CD80 include CD28, CD152 (CTLA4), and PD-L1. The cis interaction between PD-L1 and CD80 can inhibit the functions of PD-1 and CTLA-4; the binding of CD28 to CD80 can generate a costimulatory signal that enhances T cell activation, function, and survival in response to cognate antigens. Through these mechanisms, CD80 maintains a balance between T cell activation and inhibition and plays a role in attacking tumor cells.

To date, monoclonal antibodies or diabodies targeting CTLA4 or PD-L1 have been developed for these mechanisms. However, these antibodies may cause low patient response rates and drug resistance due to the lack of sufficient T cell co-stimulation in the tumor microenvironment. Targeting CD80 ligands has been an attractive strategy in cancer and autoimmunity in view of the important role of CD80 ligands in immunoregulation.

### SUMMARY

The present application provides a fusion protein comprising CD80, which is capable of binding to PD-L1, CTLA-4, and CD28, activating the activity of T lymphocytes, and inhibiting the growth and/or proliferation of a tumor or tumor cell. The present application also relates to a nucleic acid encoding the fusion protein, a vector comprising the fusion protein, a cell comprising the nucleic acid or the vector, a method for preparing the fusion protein, and use of the fusion protein.

In one aspect, the present application provides a CD80 protein variant, which comprises a functionally active fragment of a CD80 protein, or a variant thereof, wherein the variant has mutations at one or more amino acid sites as compared to a corresponding functionally active fragment of wild-type CD80, and the variant has one or more of the following properties:
a) binding to PD-L1;
b) binding to CTLA-4;
c) binding to CD28;
d) activating the activity of T lymphocytes; and
e) inhibiting the growth and/or proliferation of a tumor or tumor cell.

In certain embodiments, the PD-L1 is human PD-L1.

In certain embodiments, the CTLA-4 is human CTLA-4.

In certain embodiments, the CD28 is human CD28.

In certain embodiments, the functionally active fragment of the CD80 protein comprises an extracellular domain (ECD) of the CD80 protein, or a fragment thereof.

In certain embodiments, the fragment of the extracellular domain of the CD80 protein comprises an IgV domain of the CD80 protein.

In certain embodiments, the CD80 protein variant comprises, as compared to a corresponding extracellular domain of the wild-type CD80 protein, or a fragment thereof, amino acid mutations at one or more amino acid residues selected from: N55, N64, N152, N173, N177, N192, and N198.

In certain embodiments, the CD80 protein variant comprises mutations at any one of groups of amino acid positions selected from:
1) N64;
2) N55 and N64;
3) N55, N64, and N152;
4) N55, N64, and N192;
5) N55, N64, and N198;
6) N55, N64, N152, and N198;
7) N55, N64, N152, and N192;
8) N55, N64, N152, N173, N177, and N198;
9) N55, N64, N152, N173, and N198; and
10) N55, N64, N152, N177, and N198.

In certain embodiments, the CD80 protein variant comprises, as compared to a corresponding functionally active fragment of a wild-type CD80 protein, amino acid mutations at one or more amino acid residues selected from: N55A/Q, N64A, N152A, N173Q, N177A, N192A, and N198A/Q.

In certain embodiments, the CD80 protein variant comprises any one of groups of amino acid mutations selected from:
1) N64A;
2) N55A and N64A;
3) N55A, N64A, and N152A;
4) N55A, N64A, and N192A;
5) N55A, N64A, and N198A;
6) N55A, N64A, N152A, and N198A;
7) N55A, N64A, N152A, and N192A;
8) N55Q, N64A, N152A, and N198Q;
9) N55Q, N64A, N152A, N173Q, N177A, and N198Q;
10) N55Q, N64A, N152A, N173Q, and N198Q; and
11) N55Q, N64A, N152A, N177A, and N198Q.

In certain embodiments, the CD80 protein variant comprises at least one first-class amino acid mutation capable of maintaining or enhancing the stability of the CD80 protein variant.

In certain embodiments, the CD80 protein variant comprises first-class amino acid mutations at one or more of the following amino acid positions: N55, N64, N152, N192, and N198.

In certain embodiments, the CD80 protein variant comprises first-class amino acid mutations at any one of groups of amino acid positions selected from:
1) N64;
2) N55 and N64;
3) N55, N64, and N152;
4) N55, N64, and N192;
5) N55, N64, and N198;
6) N55, N64, N152, and N198; and
7) N55, N64, N152, and N192.

In certain embodiments, the CD80 protein variant comprises one or more of the following first-class amino acid mutations: N55A/Q, N64A, N152A, N192A, and N198A/Q.

In certain embodiments, the CD80 protein variant comprises any one of groups of first-class amino acid mutations selected from:
1) N64A;
2) N55A and N64A;
3) N55A, N64A, and N152A;
4) N55A, N64A, and N192A;
5) N55A, N64A, and N198A;
6) N55A, N64A, N152A, and N198A;
7) N55A, N64A, N152A, and N192A; and
8) N55Q, N64A, N152A, and N198Q.

In certain embodiments, the CD80 protein variant comprises at least one second-class amino acid mutation capable of retaining the binding activity of the CD80 protein variant.

In certain embodiments, the CD80 protein variant comprises second-class amino acid mutations at one or more of the following amino acid positions: N152; and N192 and N198.

In certain embodiments, the CD80 protein variant comprises one or more of the following second-class amino acid mutations: N152A; and N192A and N198A/Q.

In certain embodiments, the CD80 protein variant comprises second-class amino acid mutations at positions N55 and/or N64.

In certain embodiments, the CD80 protein variant comprises second-class amino acid mutations of N55A/Q and/or N64A.

In certain embodiments, the CD80 protein variant comprises at least one third-class amino acid mutation capable of maintaining the affinity of the CD80 protein variant.

In certain embodiments, the CD80 protein variant comprises third-class amino acid mutations at one or more of the following amino acid positions: N55, N64, N152, and N198.

In certain embodiments, the CD80 protein variant comprises one or more of the following third-class amino acid mutations: N55A/Q, N64A, N152A, and N198A.

In certain embodiments, the CD80 protein variant comprises a third-class amino acid mutation at position N192.

In certain embodiments, the CD80 protein variant comprises a third-class amino acid mutation of N192A.

In certain embodiments, the CD80 protein variant comprises at least one fourth-class amino acid mutation capable of enhancing the binding of the CD80 protein variant to CD28 and/or PD-L1.

In certain embodiments, the CD80 protein variant comprises fourth-class amino acid mutations at positions N173 and/or N177.

In certain embodiments, the CD80 protein variant comprises fourth-class amino acid mutations at N173Q and/or N177A.

In certain embodiments, numbers of the amino acid positions in the CD80 protein variant are determined with reference to position numbers of amino acids in SEQ ID NO: 1.

In certain embodiments, the CD80 protein variant comprises an amino acid sequence set forth in any one of SEQ ID NO: 5, SEQ ID NO: 6, SEQ ID NO: 8, SEQ ID NO: 9, SEQ ID NO: 10, SEQ ID NO: 19, SEQ ID NO: 20, SEQ ID NO: 21, SEQ ID NO: 22, SEQ ID NO: 23, and SEQ ID NO: 24.

In certain embodiments, the CD80 protein variant is capable of binding to PD-L1, CD28, and/or CTLA-4.

In certain embodiments, the CD80 protein variant has a sialic acid content of greater than or equal to about 0.7.

In another aspect, the present application provides a fusion protein comprising the CD80 protein variant.

In certain embodiments, the fusion protein further comprises an immunoglobulin Fc region or a variant thereof.

In certain embodiments, the CD80 protein variant and the immunoglobulin Fc region in the fusion protein are linked directly or indirectly.

In certain embodiments, the immunoglobulin Fc region in the fusion protein comprises an Fc region of an IgG.

In certain embodiments, the IgG of the fusion protein is selected from: IgG1 and IgG4.

In certain embodiments, the CD80 protein variant in the fusion protein is located at the N-terminus or C-terminus of the immunoglobulin Fc region.

In certain embodiments, the immunoglobulin Fc region in the fusion protein comprises an amino acid sequence selected from: SEQ ID NO: 25 and SEQ ID NO: 26.

In certain embodiments, the fusion protein comprises an amino acid sequence set forth in any one of SEQ ID NOs: 27-37.

In another aspect, the present application also provides a polypeptide comprising the CD80 protein variant or the fusion protein.

In another aspect, the present application provides one or more isolated nucleic acid molecules, which encode the CD80 protein variant described herein, the fusion protein described herein, or the polypeptide described herein.

In another aspect, the present application provides a vector, which comprises the nucleic acid molecules described herein.

In another aspect, the present application provides a host cell, which comprises the nucleic acid molecules described herein or the vector described herein.

In another aspect, the present application provides a method for preparing the CD80 protein variant described herein or the fusion protein described herein, which comprises culturing the cell described herein under conditions that enable the fusion protein to be expressed.

In another aspect, the present application provides a pharmaceutical composition, which comprises the CD80 variant protein described herein, the fusion protein described herein, the polypeptide described herein, the nucleic acid molecules described herein, the vector described herein, and/or the host cell described herein, and optionally a pharmaceutically acceptable carrier.

In another aspect, the present application provides a pharmaceutical combination, which comprises the CD80 protein variant and an immune checkpoint inhibitor.

In another aspect, the present application provides a pharmaceutical combination, which comprises the fusion protein and an immune checkpoint inhibitor.

In another aspect, the present application provides a pharmaceutical combination, which comprises the polypeptide and an immune checkpoint inhibitor.

In certain embodiments, the immune checkpoint comprises PD-1, PD-L1, GITR, and/or CTLA-4.

In certain embodiments, the immune checkpoint comprises one or more selected from: a PD-1 antibody, a PD-L1 antibody, a CTLA-4 antibody, and a GITR antibody.

In another aspect, the present application provides a method for modulating an immune response in a subject, which comprises administering the CD80 protein variant, the fusion protein, or the polypeptide described herein.

In certain embodiments, the method for modulating an immune response in a subject comprises increasing an immune response.

In certain embodiments, in the method for modulating an immune response in a subject, the CD80 protein variant, the fusion protein, or the polypeptide exhibits increased binding affinity for PD-L1 as compared to a wild-type CD80 protein or a fragment thereof.

In certain embodiments, in the method for modulating an immune response in a subject, the CD80 protein variant, the fusion protein, or the polypeptide exhibits increased binding affinity for CD28 as compared to a wild-type CD80 protein or a fragment thereof.

In certain embodiments, in the method for modulating an immune response in a subject, the CD80 protein variant, the fusion protein, or the polypeptide exhibits enhanced activity for activating T lymphocytes as compared to a wild-type CD80 protein or a fragment thereof.

In another aspect, the present application provides use of the CD80 protein variant, the CD80 fusion protein, the polypeptide, the nucleic acid molecules, the vector, the host cell, the pharmaceutical composition, or the pharmaceutical combination, in the preparation of a medicament for preventing and/or treating a disease and/or disorder.

In certain embodiments, the disease and/or disorder includes a tumor.

In certain embodiments, the tumor includes a solid tumor and/or a hematologic tumor.

In certain embodiments, the tumor includes a tumor that is responsive to the proliferation of central memory T cells.

In certain embodiments, the tumor in the use includes: malignant melanoma, breast cancer, gastric cancer, renal cancer, non-small cell lung cancer, colon cancer, kidney cancer, head and neck squamous cell carcinoma, liver cancer, kidney cancer, mesothelioma bladder cancer, pancreatic cancer, ovarian cancer, endometrial cancer, and lymphoma.

In another aspect, the present application provides a method for preventing and/or treating a disease and/or disorder, which comprises administering to a subject in need thereof the CD80 protein variant, the fusion protein, the polypeptide, the nucleic acid molecules, the vector, the host cell, the pharmaceutical composition, or the pharmaceutical combination.

In certain embodiments, the disease and/or disorder includes a tumor.

In certain embodiments, the tumor includes a solid tumor and/or a hematologic tumor.

In certain embodiments, the tumor includes a tumor that is responsive to the proliferation of central memory T cells.

In another aspect, the present application provides the CD80 protein variant, the fusion protein, the polypeptide, the nucleic acid molecules, the vector, the host cell, the pharmaceutical composition, or the pharmaceutical combination, which is for use in preventing and/or treating a disease and/or disorder.

In certain embodiments, the disease and/or disorder includes a tumor.

In certain embodiments, the tumor includes a solid tumor and/or a hematologic tumor.

In certain embodiments, the tumor includes a tumor that is responsive to the proliferation of central memory T cells.

In another aspect, the present application provides a method for detecting a CD80-binding ligand in a biological sample, which comprises bringing the biological sample into contact with a binding reagent for the CD80 protein variant or the fusion protein described herein.

In certain embodiments, the binding ligand in the method includes PD-L1, CD28, and/or CTLA-4. In another aspect, the present application also provides a method for detecting the frequency and/or proliferation of central memory T cells in a subject, which comprises administering the CD80 protein variant, the fusion protein, or the polypeptide.

In certain embodiments, the central memory T cells are CD95+ and CD28+ cells.

In certain embodiments, the central memory T cells are CD4+ central memory T cells and/or CD8+ central memory T cells.

In another aspect, the present application also provides a kit, which comprises the CD80 protein variant, the fusion protein, the polypeptide, the nucleic acid molecules, the vector, the host cell, the pharmaceutical composition, or the pharmaceutical combination.

In certain embodiments, the kit is for use in detecting the frequency and/or proliferation of central memory T cells in a subject.

In certain embodiments, the kit is for use in detecting the presence and/or amount of a CD80 binding ligand in a biological sample.

In another aspect, the present application also provides the CD80 protein variant, the fusion protein, the polypeptide, the nucleic acid molecules, the vector, the host cell, the pharmaceutical composition, or the pharmaceutical combination, which is for use in enhancing the proliferation or frequency of central memory T cells.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the drawings and description of the present application are provided only for purpose of illustration rather than limitation.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described as follows:
FIG. 1 shows a structural diagram of the CD80 fusion protein according to the present application.
FIGs. 2A-2B show the assay results for the activity of the CD80 fusion proteins according to the present application for binding to a human PD-L1 protein.
FIGs. 3A-3D show the assay results for the activity of the CD80 fusion proteins according to the present application for binding to a human CTLA-4 protein.
FIGs. 4A-4C show the assay results for the activity of the CD80 fusion proteins according to the present application for binding to a human CD28 protein.
FIG. 5 shows the assay results for the activity of the CD80 fusion protein according to the present application for binding to human CTLA-4 positive cells.
FIG. 6 shows the assay results for the activity of the CD80 fusion protein according to the present application for binding to human PD-L1 positive cells.
FIG. 7 shows the assay results for the activity of the CD80 fusion protein according to the present application for binding to human CD28 positive cells.
FIG. 8 shows the assay results for the activity of the CD80 fusion proteins according to the present application for activating T lymphocytes under culture conditions of aAPC/T mixed cells.
FIGs. 9A-9C show the assay of the CD28 activation activity of the CD80 fusion proteins according to the present application.
FIG. 10 shows the results for the anti-tumor activity of the CD80 fusion proteins according to the present application in a CT26 subcutaneous xenograft model.
FIG. 11 shows the results for the anti-tumor effect of the CD80 fusion proteins according to the present application in combination with a Treg inhibitor in a CT26 subcutaneous xenograft model.
FIG. 12 shows the results for the anti-tumor effect of the CD80 fusion proteins according to the present application in combination with a PD-1 inhibitor in a CT26 subcutaneous xenograft model.
FIG. 13 shows the results for the anti-tumor effect of the CD80 fusion proteins according to the present application in combination with a CTLA-4 inhibitor in a CT26 subcutaneous xenograft model.
FIGs. 14A-14B show the results for cytokine release induced by the CD80 fusion protein according to the present application.
FIG. 15 shows the results for charge heterogeneity analysis of the CD80 fusion proteins according to the present application.
FIG. 16 shows the anti-tumor effect of the CD80 fusion proteins according to the present application with varying degrees of sialic acid modification.
FIG. 17 shows the mean plasma concentration-time curves of groups after single intravenous infusions of CD80-WT-G1 and CD80M28 stock solutions in Sprague Dawley rats. (Mean + SD, n = 8)

### Examples

The implementation of the invention to which the present application relates is described below with reference to specific embodiments, and other advantages and effects of the invention to which the present application relates will be readily apparent to those skilled in the art from the disclosure of the present specification.

### Definitions of Terms

In the present application, the term "CD80 protein variant" refers to a protein having an altered amino acid sequence relative to wild-type CD80. The wild-type CD80 generally refers to naturally occurring CD80 obtained from nature. In the present application, the wild-type CD80 may be human CD80. The variant generally refers to substitutions, deletions, and/or additions of one or more amino acids of the sequence as compared to the wild type. In certain cases, the CD80 protein variant may be an extracellular domain (ECD) of a CD80 protein; further, the CD80 protein variant may be a protein that has undergone mutations at one or more amino acid residues based on ECD; in certain instances, the CD80 protein variant may be an IgV domain of a CD80 protein; further, the CD80 protein variant may be a protein that has undergone mutations at one or more amino acid residues based on the IgV domain. In certain cases, the sequence number of the human CD80 in GenBank database may be NP_005182.1.

In the present application, the term "extracellular domain (ECD) of a CD80 protein" generally refers to a polypeptide fragment located on a cell membrane surface expressing a CD80 protein. The CD80 protein is a transmembrane glycoprotein comprising two Ig-like extracellular domains, a transmembrane helix segment, and a short cytoplasmic tail. The Ig-like extracellular domain comprises single V-domain and C2-domain. The extracellular domain of the CD80 protein may comprise all or part of the polypeptide fragments of the two Ig-like extracellular domains. In the present application, the extracellular domain of the CD80 protein may refer to an extracellular domain of a human CD80 protein. For example, the extracellular domain of the CD80 protein described herein may be a protein domain comprising all or part of an amino acid sequence set forth in SEQ ID NO: 1.

In the present application, the term "IgV domain of a CD80 protein" generally refers to a polypeptide fragment of a V-domain located on a cell membrane surface expressing a CD80 protein. The IgV domain of the CD80 protein may comprise all or part of the polypeptide fragment of the V-domain. In the present application, the IgV domain of the CD80 protein may refer to an IgV domain of a human CD80 protein. For example, the IgV domain of the CD80 protein described herein may be a protein domain comprising all or part of an amino acid sequence set forth in SEQ ID NO: 2.

In the present application, the terms "PD-L1" and "PDL1" are used interchangeably and generally refer to programmed cell death ligand 1, which may also be referred to as surface antigen cluster of differentiation 274 (CD274) or B7 homolog 1 (B7-H1), PDCD1L1, PDCD1LG1, or PDL1, and belongs to the tumor necrosis factor superfamily. "PD-L1" may be intact PD-L1 and fragments thereof, or may be functional variants, isotypes, species homologs, derivatives, and analogs of PD-L1, and analogs sharing at least one common epitope with PD-L1. PD-L1 may be a type I transmembrane glycoprotein consisting of 290 amino acid residues, which comprises an IgV-like region, an IgC-like region, a transmembrane hydrophobic region, and an intracellular tail of 30 amino acids. In the present application, the PD-L1 may be human PD-L1.

In the present application, the term "CTLA-4" generally refers to cytotoxic T-lymphocyte antigen-4, which may also be referred to as CTLA4 or surface antigen cluster of differentiation 152 (CD152), is a leukocyte differentiation antigen sharing a B7 molecular ligand with CD28, and is an immunosuppressive receptor belonging to the CD28 family. CTLA-4 comprises an extracellular V-domain, a transmembrane domain, and a cytoplasmic tail. "CTLA-4" may be intact CTLA-4 and fragments thereof, or may be functional variants, isotypes, species homologs, derivatives, and analogs of CTLA-4, and analogs sharing at least one common epitope with CTLA-4. In the present application, the CTLA-4 may be human CTLA-4.

In the present application, the term "CD28" refers generally to surface antigen cluster of differentiation 28, which is a costimulatory molecule expressed on the surface of T lymphocytes, and a receptor for CD80 and CD86 proteins, and mediates the co-stimulation of T cells and promotes their survival, proliferation and cytokine production. "CD28" may be intact CD28 and fragments thereof, or may be functional variants, isotypes, species homologs, derivatives, and analogs of CD28, and analogs sharing at least one common epitope with CD28. In the present application, the CD28 may be human CD28.

In the present application, the term "amino acid mutation" generally refers to amino acid substitution, deletion, insertion, and modification. Any combination of substitutions, deletions, insertions, and modifications may be made to arrive at the final construct. In the present application, the amino acid mutation may be replacement of at least one existing amino acid residue with another different amino acid residue (an alternative amino acid residue). The alternative amino acid residue may be a "naturally occurring amino acid residue" and is selected from the group consisting of: alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L)), lysine (lys, K), methionine (met, M), phenylalanine (phe, F), proline (pro, P), serine (ser, S), threonine (thr, T), tryptophan (trp, W), tyrosine (tyr, Y), and valine (val, V).

In the present application, the amino acid positions are confirmed based on the amino acid sequence set forth in SEQ ID NO: 1 (the amino acid sequence of the extracellular domain of human CD80 (UniProtKB/Swiss-Prot: P33681.1 sequence), i.e., positions 35-243 of the UniProtKB/Swiss-Prot: P33681.1 sequence). That is, the first amino acid in the amino acid sequence set forth in SEQ ID NO: 1 is numbered as the first amino acid.

In the present application, the extracellular domain of the CD80 protein may comprise amino acids at positions 35-243 of the UniProtKB/Swiss-Prot: P33681.1 sequence.

In the present application, the IgV domain of the CD80 protein may comprise amino acids at positions 35-140 of the UniProtKB/Swiss-Prot: P33681.1 sequence.

In the present application, the term "first-class amino acid mutation" generally refers to an amino acid mutation that has an influence on the stability of the CD80 protein variant. The stability of the CD80 protein variant refers to the ability of the CD80 protein variant to maintain the structure and function approximately consistent with that of the CD80 protein before mutation, and the influence may be the maintenance, reduction, and enhancement of the stability of the CD80 protein variant. In the present application, the CD80 protein variant may have an amino acid sequence mutation based on the human CD80 protein. For example, in the present application, the first-class amino acid mutation may include one or more amino acid mutations at all or part of a fragment of AA35-AA242 of human CD80, and in certain cases, the one or more amino acid mutations may include an amino acid mutation at any one of positions selected from: N55, N64, N152, N192, and N198.

In the present application, the term "second-class amino acid mutation" generally refers to an amino acid mutation that has an influence on the binding activity of the CD80 protein variant. The influence may be the maintenance, reduction, and enhancement of the binding activity of the CD80 protein variant. In the present application, the CD80 protein variant may have an amino acid sequence mutation based on the human CD80 protein. For example, in the present application, the second-class amino acid mutation may include one or more amino acid mutations at all or part of a fragment of AA35-AA242 of human CD80, and in certain cases, the one or more amino acid mutations may include an amino acid mutation at any one of positions selected from: N55, N64, N152, N192, and N198.

In the present application, the term "binding activity" generally refers to the ability of two binding sites to bind to each other, the binding site may be a region on a binding molecule, and the binding molecule may include a protein, an enzyme substrate, a second messenger, a hormone, or an allosteric modulator. In the present application, the binding activity may be the ability of the CD80 protein variant to bind to its ligand, and the binding may be either reversibly (transiently and non-covalently) or covalently reversibly or irreversibly.

In the present application, the term "third-class amino acid mutation" generally refers to an amino acid mutation that has an influence on the affinity of the CD80 protein variant. The influence may be the maintenance, reduction, and enhancement of the affinity of the CD80 protein variant. In the present application, the CD80 protein variant may have an amino acid sequence mutation based on the human CD80 protein. For example, in the present application, the third-class amino acid mutation may include one or more amino acid mutations at all or part of a fragment of AA35-AA242 of human CD80, and in certain cases, the one or more amino acid mutations may include an amino acid mutation at any one of positions selected from: N55, N64, N152, N192, and N198.

In the present application, the term "affinity" generally refers to the specific affinity ability to cause the interaction of two or more substances, and in certain cases, it may be the affinity ability of a receptor to a ligand.

In the present application, the term "fourth-class amino acid mutation" generally refers to an amino acid mutation that has an influence on the binding of the CD80 protein variant to CD28 and/or PD-L1. The influence may be the maintenance, reduction, and enhancement of the binding of the CD80 protein variant to CD28 and/or PD-L1. In the present application, the CD80 protein variant may have an amino acid sequence mutation based on the human CD80 protein. For example, in the present application, the third-class amino acid mutation may include one or more amino acid mutations at all or part of a fragment of AA35-AA242 of human CD80, and in certain cases, the one or more amino acid mutations may include an amino acid mutation at any one of positions selected from: N173 and/or N177.

In the present application, the term "fusion protein" generally refers to a macromolecule formed by linking two or more different protein molecules by chemical and/or genetic fusion. The fusion protein may be an expression product obtained after recombination of two or more genes by recombinant DNA techniques, or a group of proteins mediating the fusion of two or more cell plasma membranes. In the present application, the fusion protein may be a protein molecule, which is expressed in a eukaryotic or prokaryotic cell after linking a gene of interest to a gene of the immunoglobulin Fc region or the variant fragment thereof at the gene level, and has the above two domains.

In the present application, the term "immunoglobulin Fc region" generally refers to a crystallizable segment of an immunoglobulin, which may include CH2 and CH3 domains of immunoglobulins IgG, IgA, and IgD, or fragments thereof, or may include CH2, CH3, and CH4 domains of IgM and IgE, or fragments thereof. For example, the IgG may include IgG1, IgG2, IgG3, or IgG4.

The terms "polypeptide", "peptide", and "protein" are used interchangeably herein and refer to a polymer of amino acid residues. This term may be used to refer to an amino acid polymer in which one or more amino acid residues are artificially synthesized chemical mimetics of their corresponding naturally occurring amino acids, or to naturally occurring amino acid polymers, amino acid polymers containing modified residues, and non-naturally occurring amino acid polymers.

In the present application, the term "nucleic acid molecule" generally refers to nucleotides, deoxyribonucleotides, or ribonucleotides, or analogs thereof in an isolated form and of any length that are isolated from its natural environment or artificially synthesized.

In the present application, the term "vector" generally refers to a nucleic acid molecule capable of self-replication in a suitable host, which transfers an inserted nucleic acid molecule into a cell (e.g., host cell) and/or between host cells. The vector may include a vector primarily used for inserting DNA or RNA into a cell, a vector primarily used for replicating DNA or RNA, and a vector primarily used for expressing transcription and/or translation of DNA or RNA. The vector also includes vectors having a variety of the above functions. The vector may be a polynucleotide capable of transcription and translation into a polypeptide when introduced into a suitable cell. Generally, the vector can produce the desired expression product by culturing a suitable cell comprising the vector. In the present application, the vector may be a plasmid.

In the present application, the term "pharmaceutical composition" generally refers to a composition suitable for administration to a patient, which may be a human patient. For example, the pharmaceutical composition described herein may comprise the CD80 variant protein described herein, the fusion protein described herein, the nucleic acid molecules described herein, the vector described herein, and/or the cell described herein, and optionally a pharmaceutically acceptable carrier. Furthermore, the pharmaceutical composition may further comprise suitable formulations of one or more (pharmaceutically effective) carriers, stabilizers, excipients, diluents, solubilizers, surfactants, emulsifiers, and/or preservatives. The acceptable ingredients of the composition are preferably non-toxic to the recipient at the dose and concentration used. The pharmaceutical composition of the present invention includes, but is not limited to, liquid, frozen, and lyophilized compositions.

In the present application, the term "pharmaceutical combination" generally refers to the use of two or more drugs in combination. In the pharmaceutical combination, different pharmaceutical components may be placed in a mixed manner or in a separated manner. In the pharmaceutical combination, different pharmaceutical components may be placed in the same container or in different containers. In the pharmaceutical combination, different pharmaceutical components may be administered simultaneously or separately. For example, different pharmaceutical components may be administered sequentially. In the pharmaceutical combination, different pharmaceutical components may be administered in the same manner or in different manners.

In the present application, the term "pharmaceutically acceptable carrier" generally refers to any and all solvents, dispersion media, coatings, isotonic and absorption delaying agents, and the like, that are compatible with pharmaceutical administration, are generally safe and non-toxic, and are neither biologically nor otherwise undesirable.

In the present application, the term "tumor" generally refers to a neoplasm or solid lesion formed by abnormal cell growth. In the present application, the tumor may be a solid tumor or a non-solid tumor. In certain cases, the tumor may include a tumor that is responsive to the proliferation of central memory T cells. In certain cases, the tumor may include a tumor comprising cells that are surfacepositive for CTLA-4.

In the present application, the protein, polypeptide, and/or amino acid sequence involved is also to be understood as including at least the following ranges: variants or homologs having the same or similar function as the protein or polypeptide.

In the present application, the variants may be, for example, proteins or polypeptides obtained by substitution, deletion, or addition of one or more amino acids in the amino acid sequence of the protein and/or polypeptide (e.g., the CD80 protein variant). For example, the functional variants may comprise proteins or polypeptides with amino acid changes by substitutions, deletions and/or insertions of at least 1 (for example, 1-30, 1-20 or 1-10, for another example, 1, 2, 3, 4 or 5) amino acid. The functional variants may substantially retain the biological properties of the protein or the polypeptide before the changes (e.g., substitutions, deletions, or additions). For example, the functional variants may retain at least 60%, 70%, 80%, 90% or 100% of the biological activity (e.g., antigen-binding ability) of the protein or the polypeptide before the changes. For example, the substitutions may be conservative.

In the present application, the homologs may be proteins or polypeptides comprising an amino acid sequence having at least about 85% (e.g., having at least about 85%, about 90%, about 91%, about 92%, about 93%, about 94%, about 95%, about 96%, about 97%, about 98%, about 99%, or higher) sequence homology to the amino acid sequence of the protein and/or the polypeptide (e.g., the CD80 protein variant).

In the present application, the homology generally refers to similarity, likeness, or association between two or more sequences. "Percent sequence homology" can be calculated by the following steps: comparing two sequences to be aligned in a comparison window; determining the number of positions at which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to give the number of matched positions; dividing the number of matched positions by the total number of positions in the comparison window (i.e., the window size); and multiplying the result by 100 to give a percent sequence homology. Alignment for determining the percent sequence homology can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithm necessary to yield optimal alignment within a full-length sequence range or target sequence region being compared. The homology can also be determined by the following methods: FASTA and BLAST. For the description of the FASTA algorithm, see W. R. Pearson and D. J. Lipman, "Improved Tools for Biological Sequence Comparison" (Proc. Natl. Acad. Sci.), 85: 2444-2448, 1988; and D. J. Lipman and W. R. Pearson, "Rapid and Sensitive Protein Similarity Searches", Science, 227: 1435-1441, 1989. For description of the BLAST algorithm, see S. Altschul, W. Gish, W. Miller, E. W. Myers and D. Lipman, "ABasic Local Alignment Search Tool", Journal of Molecular Biology, 215: 403-410, 1990.

In the present application, the term "comprise" or "comprising" generally means including, containing, or encompassing. In certain cases, the term also means "being" or "consisting of...".

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### DETAILED DESCRIPTION OF THE INVENTION

### CD80 Protein Variant

In one aspect, the present application provides a CD80 protein variant, which may comprise a functionally active fragment of a CD80 protein, or a variant thereof, wherein the variant has mutations at one or more amino acid sites as compared to a corresponding functionally active fragment of wild-type CD80.

In the present application, the CD80 protein variant may have one or more of the following properties: 1) binding to PD-L1; 2) binding to CTLA-4; 3) binding to CD28; 4) activating the activity of T lymphocytes; and 5) inhibiting the growth and/or proliferation of a tumor or tumor cell.

For example, the CD80 protein variant is capable of binding to PD-L1 and CD28 proteins simultaneously. For example, the CD80 variant is capable of binding to PD-L1, CD28, and CTLA-4 proteins simultaneously.

In the present application, the PD-L1 may be human PD-L1. In the present application, the CD28 may be human CD28. In the present application, the CTLA-4 may be human CTLA-4.

In the present application, the CD80 protein may be a human CD80 protein.

In the present application, the CD80 protein variant may comprise a functionally active fragment of CD80, or a variant thereof. For example, the functionally active fragment of CD80 may comprise an extracellular domain of the CD80 protein, or a fragment thereof. For example, the fragment of the extracellular domain of the CD80 protein may comprise an IgV domain of CD80.

In the present application, an IgV domain of a wild-type CD80 protein may comprise an amino acid sequence set forth in SEQ ID NO: 2.

In the present application, an extracellular domain of a wild-type CD80 protein may comprise an amino acid sequence set forth in SEQ ID NO: 1.

In the present application, the variant of the IgV domain of the CD80 protein may comprise one or more amino acid mutations.

In the present application, the variant of the IgV domain of the CD80 protein may comprise the following amino acid sequence: VIHVTKEVKEVATLSCGHNVSVEELAQTRIYWQKEKKM VLTMMSGDMNIWPEYKNRTIFDITNX₁LSIVILALRPSDEGTYECVVLKYEKDAFKREHLAE VTLSVKA. The variant of the IgV domain of the CD80 protein may comprise at least an amino acid substitution at X₁.

In the present application, the variant of the IgV domain of the CD80 protein may comprise at least an amino acid substitution at X₁ as compared to the IgV domain of the CD80 protein comprising set forth in SEQ ID NO: 2. For example, the amino acid at X₁ may be substituted with A.

In the present application, the variant of the extracellular domain of the CD80 protein may comprise one or more amino acid mutations.

In the present application, the variant of the extracellular domain of the CD80 protein may comprise the following amino acid sequence: VIHVTKEVKEVATLSCGHX₁VSVEELAQTRIYWQKE KKMVLTMMSGDMNIWPEYKX₂RTIFDITNX₃LSIVILALRPSDEGTYECVVLKYEKDAFKRE HLAEVTLSVKADFPTPSISDFEIPTSNIRRIICSTSGGFPEPHLSWLENGEELNAIX₄TTVSQDP ETELYAVSSKLDFX₅MTTX₆HSFMCLIKYGHLRVX₇QTFNWX₈TTKQEHPDNL. The variant of the extracellular domain of the CD80 protein may comprise at least an amino acid substitution at X₁, X₂, X₃, X₄, X₅, X₆, X₇, and/or X₈.

For example, the CD80 protein variant may comprise amino acid mutations at one or more of positions X₁, X₂, X₃, X₄, X₅, X₆, X₇, and X₈. The amino acid at X₁ may be substituted with A; the amino acid at X₂ may be substituted with A or Q; the amino acid at X₃ may be substituted with A; the amino acid at X₄ may be substituted with A; the amino acid at X₅ may be substituted with A or Q; the amino acid at X₆ may be substituted with A; the amino acid at X₇ may be substituted with A; the amino acid at X₈ may be substituted with A or Q.

In the present application, the CD80 protein variant may comprise a first-class amino acid mutation. For example, the first-class amino acid mutation is capable of maintaining the stability of the CD80 protein variant.

In the present application, in the CD80 protein variant comprising the first-class amino acid mutation, the variant of the extracellular domain of the CD80 protein may comprise at least amino acid substitutions at X₂, X₃, X₄, X₇, and/or X₈ as compared to the extracellular domain of the CD80 protein comprising set forth in SEQ ID NO: 1, wherein the amino acid at X₂ may be substituted with A or Q; the amino acid at X₃ may be substituted with A; the amino acid at X₄ may be substituted with A; the amino acid at X₇ may be substituted with A; the amino acid at X₈ may be substituted with A or Q.

For example, the first-class amino acid mutation may include an amino acid mutation at position N64. In the present application, the first-class amino acid mutation may include amino acid mutations at positions N55 and N64. For example, the first-class amino acid mutation may include amino acid mutations at positions N55, N64, and N152. For example, the first-class amino acid mutation may include amino acid mutations at positions N55, N64, and N192. For example, the first-class amino acid mutation may include amino acid mutations at positions N55, N64, and N198. For example, the first-class amino acid mutation may include amino acid mutations at positions N55, N64, N152, and N198. For example, the first-class amino acid mutation may include amino acid mutations at positions N55, N64, N152, and N192.

For example, the first-class amino acid mutation may include an amino acid mutation of N64A. For example, the first-class amino acid mutation may include amino acid mutations of N55A and N64A. For example, the first-class amino acid mutation may include amino acid mutations of N55A, N64A, and N152A. For example, the first-class amino acid mutation may include amino acid mutations of N55A, N64A, and N192A. For example, the first-class amino acid mutation may include amino acid mutations ofN55A, N64A, and N198A. For example, the first-class amino acid mutation may include amino acid mutations of N55A, N64A, N152A, and N198A. For example, the first-class amino acid mutation may include amino acid mutations of N55A, N64A, N152A, and N192A. For example, the first-class amino acid mutation may include amino acid mutations of N55Q N64A, N152A, and N198Q.

In the present application, the CD80 variant may comprise a second-class amino acid mutation. In the present application, the second-class amino acid mutation is capable of maintaining the binding activity of the CD80 protein variant.

In the present application, in the CD80 protein variant comprising the second-class amino acid mutation, the variant of the extracellular domain of the CD80 protein may comprise at least amino acid substitutions at X₄, X₇, and/or X₈ as compared to the extracellular domain of the CD80 protein comprising set forth in SEQ ID NO: 1, wherein the amino acid at X₄ may be substituted with A; the amino acid at X₇ may be substituted with A; the amino acid at X₈ may be substituted with A and Q.

For example, the second-class amino acid mutation may include mutations at one or more of amino acid positions N152, N192, and N198. For example, the second-class amino acid mutation may include one or more amino acid mutations of N152A; and N192A and N198A/Q. For example, the second-class amino acid mutation may further include amino acid mutations at positions N55 and/or N64. For example, the second-class amino acid mutation may further include amino acid mutations of N55A/Q and/or N64A.

In the present application, the CD80 protein variant may comprise a third-class amino acid mutation. In the present application, the third-class amino acid mutation is capable of maintaining the affinity of the CD80 protein variant.

In the present application, in the CD80 protein variant comprising the third-class amino acid mutation, the variant of the extracellular domain of the CD80 protein may comprise at least amino acid substitutions at X₂, X₃, X₄, and/or X₈ as compared to the extracellular domain of the CD80 protein comprising set forth in SEQ ID NO: 1, wherein the amino acid at X₂ may be substituted with A or Q; the amino acid at X₃ may be substituted with A; the amino acid at X₄ may be substituted with A; the amino acid at X₈ may be substituted with A.

For example, the third-class amino acid mutation may include amino acid mutations at one or more of the following positions: N55, N64, N152, and N198. For example, the third-class amino acid mutation may include one or more of the following amino acid mutations: N55A/Q, N64A, N152A, and N198A. For example, the third-class amino acid mutation may further include an amino acid mutation at position N192. For example, the third-class amino acid mutation may further include an amino acid mutation of N192A.

In the present application, the CD80 protein variant may comprise a fourth-class amino acid mutation. In the present application, the fourth-class amino acid mutation is capable of enhancing the binding of the CD80 protein variant to CD28 and/or PD-L1.

In the present application, in the CD80 protein variant comprising the fourth-class amino acid mutation, the variant of the extracellular domain of the CD80 protein may comprise at least amino acid substitutions at X₅ and/or X₆ as compared to the extracellular domain of the CD80 protein comprising set forth in SEQ ID NO: 1, wherein the amino acid at X₅ may be substituted with Q; the amino acid at X₆ may be substituted with A.

For example, the fourth-class amino acid mutation may include amino acid mutations at positions N173 and/or N177. For example, the fourth-class amino acid mutation may include amino acid mutations of N173Q and/or N177A.

In the present application, the CD80 protein variant may comprise an amino acid mutation at position N64 as compared to the amino acid sequence set forth in SEQ ID NO: 2. In the present application, the CD80 protein variant may comprise amino acid mutations at positions N64 and N55 as compared to the amino acid sequence set forth in SEQ ID NO: 1. In the present application, the CD80 protein variant may comprise amino acid mutations at positions N55, N64, and N152 as compared to the amino acid sequence set forth in SEQ ID NO: 1. In the present application, the CD80 protein variant may comprise amino acid mutations at positions N55, N64, and N192 as compared to the amino acid sequence set forth in SEQ ID NO: 1. In the present application, the CD80 protein variant may comprise amino acid mutations at positions N55, N64, and N198 as compared to the amino acid sequence set forth in SEQ ID NO: 1. In the present application, the CD80 protein variant may comprise amino acid mutations at positions N55, N64, N152, and N198 as compared to the amino acid sequence set forth in SEQ ID NO: 1. In the present application, the CD80 protein variant may comprise amino acid mutations at positions N55, N64, N152, and N192 as compared to the amino acid sequence set forth in SEQ ID NO: 1. In the present application, the CD80 protein variant may comprise amino acid mutations at positions N55, N64, N152, N173, N177, and N198 as compared to the amino acid sequence set forth in SEQ ID NO: 1. In the present application, the CD80 protein variant may comprise amino acid mutations at positions N55, N64, N152, N173, and N198 as compared to the amino acid sequence set forth in SEQ ID NO: 1. In the present application, the CD80 protein variant may comprise amino acid mutations at positions N55, N64, N152, N177, and N198 as compared to the amino acid sequence set forth in SEQ ID NO: 1.

In the present application, the CD80 protein variant may comprise an amino acid mutation of N64A as compared to the amino acid sequence set forth in SEQ ID NO: 1. In the present application, the CD80 protein variant may comprise amino acid mutations of N55A and N64A as compared to the amino acid sequence set forth in SEQ ID NO: 1. In the present application, the CD80 protein variant may comprise amino acid mutations of N55A, N64A, and N152A as compared to the amino acid sequence set forth in SEQ ID NO: 1. In the present application, the CD80 protein variant may comprise amino acid mutations of N55A, N64A, and N192A as compared to the amino acid sequence set forth in SEQ ID NO: 1. In the present application, the CD80 protein variant may comprise amino acid mutations of N55A, N64A, and N198A as compared to the amino acid sequence set forth in SEQ ID NO: 1. In the present application, the CD80 protein variant may comprise amino acid mutations of N55A, N64A, N152A, and N198A as compared to the amino acid sequence set forth in SEQ ID NO: 1. In the present application, the CD80 protein variant may comprise amino acid mutations of N55A, N64A, N152A, and N192A as compared to the amino acid sequence set forth in SEQ ID NO: 1. In the present application, the CD80 protein variant may comprise amino acid mutations of N55Q, N64A, N152, and N198Q as compared to the amino acid sequence set forth in SEQ ID NO: 1. In the present application, the CD80 protein variant may comprise amino acid mutations of N55Q, N64A, N152A, N173Q, N177A, and N198Q as compared to the amino acid sequence set forth in SEQ ID NO: 1. In the present application, the CD80 protein variant may comprise amino acid mutations of N55Q, N64A, N152A, N173Q, and N198Q as compared to the amino acid sequence set forth in SEQ ID NO: 1. In the present application, the CD80 protein variant may comprise amino acid mutations of N55Q, N64A, N152A, N177A, and N198Q as compared to the amino acid sequence set forth in SEQ ID NO: 1.

In the present application, the CD80 protein variant may comprise an amino acid sequence set forth in any one of SEQ ID NO: 5, SEQ ID NO: 6, SEQ ID NO: 8, SEQ ID NO: 9, SEQ ID NO: 10, SEQ ID NO: 19, SEQ ID NO: 20, SEQ ID NO: 21, SEQ ID NO: 22, SEQ ID NO: 23, and SEQ ID NO: 24.

In the present application, the CD80 protein variant may have a sialic acid content of greater than or equal to about 0.7, greater than or equal to about 0.8, greater than or equal to about 0.9, greater than or equal to about 1.0, greater than or equal to about 1.1, greater than or equal to about 1.2, greater than or equal to about 1.3, greater than or equal to about 1.4, greater than or equal to about 1.5, greater than or equal to about 2, greater than or equal to about 3, greater than or equal to about 4, greater than or equal to about 5, greater than or equal to about 6, greater than or equal to about 7, greater than or equal to about 8, greater than or equal to about 9 5, greater than or equal to about 10, greater than or equal to about 11, greater than or equal to about 12, greater than or equal to about 13, or greater than or equal to about 14.

In the present application, the CD80 protein variant may have a sialic acid content of greater than or equal to about 0.7 and less than or equal to about 15, greater than or equal to about 0.8 and less than or equal to about 15, greater than or equal to about 0.9 and less than or equal to about 15, greater than or equal to about 1.0 and less than or equal to about 15, greater than or equal to about 1.1 and less than or equal to about 15, greater than or equal to about 1.2 and less than or equal to about 15, greater than or equal to about 1.3 and less than or equal to about 15, greater than or equal to about 1.4 and less than or equal to about 15, greater than or equal to about 1.5 and less than or equal to about 15, greater than or equal to about 2 and less than or equal to about 15, greater than or equal to about 3 and less than or equal to about 15, greater than or equal to about 4 and less than or equal to about 15, greater than or equal to about 5 and less than about 15, greater than or equal to about 6 and less than or equal to about 15, greater than or equal to about 7 and less than about 15, greater than or equal to about 8 and less than about 15, greater than or equal to about 9 and less than or equal to about 15, greater than or equal to about 10 and less than or equal to about 15, greater than or equal to about 11 and less than or equal to about 15, greater than or equal to about 12 and less than or equal to about 15, greater than or equal to about 13 and less than or equal to about 15, or greater than or equal to about 14 and less than or equal to about 15.

### Fusion Protein

In another aspect, the present application provides a fusion protein comprising a CD80 protein variant. In the present application, the fusion protein may further comprise other functional fragments.

For example, the fusion protein may comprise the CD80 protein variant and an immunoglobulin Fc region or a variant thereof.

In the present application, the immunoglobulin Fc region may comprise an Fc region of an IgG. The IgG may be a human IgG, and the IgG may be a human IgG1 and/or human IgG4. The variant may be all or part of an amino acid fragment of the immunoglobulin Fc region. For example, the immunoglobulin Fc region may comprise an amino acid sequence selected from: SEQ ID NO: 25 or SEQ ID NO: 26. For example, the IgG1 Fc region may comprise an amino acid sequence set forth in SEQ ID NO: 25. For example, the IgG4 Fc region may comprise an amino acid sequence set forth in SEQ ID NO: 26.

In the present application, the CD80 protein variant and the immunoglobulin Fc region may be linked directly or indirectly. The indirect linkage described herein includes linkage via a polypeptide linker. In the present application, the polypeptide linker may comprise an amino acid sequence consisting of one or more amino acid residues. The amino acid residue may include proline or cysteine, the linker may link two binding fragments, and the binding fragment may include identical or different fragments. For example, the linker may link the CD80 protein variant and the immunoglobulin Fc region. For example, the linker may comprise an amino acid sequence of (GGGGS)n, where n may be any positive integer of 1-10. For example, n may be 1-4. For example, the linker may comprise an amino acid sequence of (GGGGS)₂. For example, the linker may comprise an amino acid sequence of (GGGGS)₃. For example, the linker may comprise an amino acid sequence of (GGGGS)₄.

In the present application, the fusion protein may comprise a first polypeptide chain or a second polypeptide chain.

In the present application, a polypeptide fragment of the first polypeptide chain may be, in order from the N-terminus to the C-terminus: CD80 protein variant-linker-immunoglobulin Fc region, wherein the CD80 protein variant may be linked to the linker, and the linker may be linked to the N-terminus of the immunoglobulin Fc region.

In the present application, a polypeptide fragment of the first polypeptide chain may be, in order from the N-terminus to the C-terminus: CD80 protein variant-immunoglobulin Fc region, wherein the CD80 protein variant may be linked to the N-terminus of the immunoglobulin Fc region.

In the present application, the fusion protein may comprise two identical first polypeptide chains, or may comprise two identical second polypeptide chains.

For example, the first polypeptide chain or the second polypeptide chain may comprise a sequence of the CD80 protein variant set forth in any one of SEQ ID NO: 5, SEQ ID NO: 6, SEQ ID NO: 8, SEQ ID NO: 9, SEQ ID NO: 10, SEQ ID NO: 19, SEQ ID NO: 20, SEQ ID NO: 21, SEQ ID NO: 22, SEQ ID NO: 23, and SEQ ID NO: 24. For example, the first polypeptide chain or the second polypeptide chain may comprise an amino acid sequence set forth in any one of SEQ ID NOs: 27-37. In the present application, the fusion protein comprises the CD80 protein variant and other protein/polypeptide molecules. For example, the other protein/polypeptide molecules do not affect the function of the CD80 protein variant.

### Nucleic Acids, Vectors, Host Cells, and Preparation Methods

In another aspect, the present application also provides one or more isolated nucleic acid molecules, which can encode the CD80 protein variant and the fusion protein thereof described herein. For example, each of the one or more nucleic acid molecules may encode the intact CD80 protein variant and the fusion protein thereof, or may encode a portion thereof.

The nucleic acid molecules described herein may be isolated. For example, they may be produced or synthesized by the following methods: (1) *in vitro* amplification, such as amplification by polymerase chain reaction (PCR); (2) cloning and recombination; (3) purification, such as separation by enzymatic cleavage and gel electrophoresis fractionation; or (4) synthesis, such as chemical synthesis. For example, the isolated nucleic acids may be produced by amplification by polymerase chain reaction (PCR). In the present application, the nucleic acids encoding the CD80 protein variant and the fusion protein thereof may be prepared by a variety of methods known in the art.

In another aspect, the present application provides one or more vectors, which comprise the one or more nucleic acid molecules described herein. Each of the vectors may comprise one or more of the nucleic acid molecules. In addition, the vectors may further comprise other genes, such as marker genes that allow selection of the vectors in suitable host cells and under suitable conditions. In addition, the vectors may comprise a variety of elements that control expression, including, but not limited to, promoter sequences, transcription initiation sequences, enhancer sequences, selection elements, and reporter genes. In addition, the vectors may further comprise an origin of replication. In addition, the vectors may include, for example, plasmids, cosmids, viruses, phages, or other vectors commonly used in, for example, genetic engineering.

In another aspect, the present application provides host cells, which may comprise the one or more nucleic acid molecules described herein and/or the one or more vectors described herein. In certain embodiments, each type of or each of the host cells may comprise one or one type of nucleic acid molecule or vector described herein. In certain embodiments, each type of or each of the host cells may comprise multiple (e.g., two or more) or multiple types (e.g., two or more types) of nucleic acid molecules or vectors described herein. For example, the vectors described herein can be introduced into the host cells, e.g., prokaryotic cells (e.g., bacterial cells), CHO cells, NS/0 cells, HEK293 cells, or other eukaryotic cells, such as plant-derived cells or fungal or yeast cells. The vectors described herein can be introduced into the host cells described herein based on methods known in the art, e.g., electroporation, lipofectine transfection, lipofectamin transfection, etc.

In another aspect, the present application provides methods for preparing the CD80 protein variant and the fusion protein thereof described herein. The methods may comprise culturing the host cells described herein under conditions that enable the CD80 protein variant and the fusion protein thereof to be expressed, for example, by adopting a suitable culture medium, a suitable temperature, a suitable culture time, etc. These methods are known to those of ordinary skill in the art.

In certain cases, the methods may further comprise a step of harvesting (e.g., isolating and/or purifying) the CD80 protein variant and the fusion protein thereof described herein. For example, affinity chromatography can be performed with protein G-agarose, protein A-agarose, or metal chelation; the CD80 protein variant and the fusion protein thereof described herein can also be purified and isolated by gel electrophoresis and/or high performance liquid chromatography, etc.

### Pharmaceutical Composition, Pharmaceutical Combination, and Use

In another aspect, the present application provides a pharmaceutical composition, which may comprise the CD80 protein variant and the fusion protein thereof, the nucleic acid molecules, the vector, or the host cell described herein, and optionally a pharmaceutically acceptable carrier.

The pharmaceutically acceptable carrier may include a buffer, an antioxidant, a preservative, a low molecular weight polypeptide, a protein, a hydrophilic polymer, an amino acid, a sugar, a chelating agent, a counter ion, a metal complex, and/or a nonionic surfactant, etc.

In the present application, the pharmaceutical composition may be formulated for oral administration, intravenous administration, intramuscular administration, *in situ* administration at the tumor site, inhalation, rectal administration, vaginal administration, transdermal administration, or administration via a subcutaneous depot. The pharmaceutical composition can be used for inhibiting tumor growth. For example, the pharmaceutical composition of the present application can inhibit or delay the development or progression of a disease, can reduce the size of a tumor (even substantially eliminate the tumor), and/or can alleviate and/or stabilize the disease state.

The pharmaceutical composition described herein may comprise a prophylactically and/or therapeutically effective amount of the CD80 protein variant and the fusion protein thereof. The prophylactically and/or therapeutically effective amount is the dose required to prevent and/or treat (at least partially treat) a disease or disorder and/or any complication thereof in a subject suffering from or at risk of developing the disease or disorder.

In another aspect, the present application provides a pharmaceutical combination, which may comprise the CD80 protein variant and an immune checkpoint inhibitor.

In another aspect, the present application provides a pharmaceutical combination, which may comprise the fusion protein and an immune checkpoint inhibitor.

In the present application, the immune checkpoint may include PD-1, PD-L1, GITR, and/or CTLA-4. In the present application, the immune checkpoint inhibitor may include one or more selected from: a PD-1 antibody, a PD-L1 antibody, a CTLA-4 antibody, and a GITR antibody.

In the present application, the PD-1 antibody may be selected from: nivolumab, palizumab, toripalimab, sintilimab, camrelizumab, and genolimzumab.

In the present application, the PD-L1 antibody may be selected from: durvalumab, atezolizumab, envafolimab, adebrelimab, sugemalimab, and tagitanlimab.

In the present application, the CTLA-4 antibody may be selected from: ipilimumab and tremelimumab.

In the present application, the GITR antibody may be selected from: MK-4166, TRX-518, INCAGN01876, BMS-986156, AMG228, IBI102, and ASP-1951.

The above antibodies are only examples, and such antibodies can be used in the present application as long as they are functionally capable of acting as inhibitors of the corresponding immune checkpoints.

In the pharmaceutical combination of the present application, the CD80 protein variant/fusion protein may be present in the same container as the immune checkpoint inhibitor. In the pharmaceutical combination of the present application, the CD80 protein variant/fusion protein may be present in a different container from the immune checkpoint inhibitor.

In the present application, the CD80 protein variant/fusion protein and the immune checkpoint inhibitor may be administered simultaneously. The simultaneous administration may be coadministration of a mixture of the components or separate administration. They may be administered in the same manner, e.g., to the same vein or other vessel, or in different manners, e.g., by intravenous administration and intratumoral administration simultaneously.

In the present application, the CD80 protein variant/fusion protein and the immune checkpoint inhibitor may be administered sequentially. The order of administration may be first the administration of the CD80 protein variant/fusion protein and then the administration of the immune checkpoint inhibitor; or first the administration of the immune checkpoint inhibitor and then the CD80 protein variant/fusion protein. They may be administered in the same manner or in different manners. The components may be administered once or in multiple times. In certain embodiments, the sequential administration may be administration at an interval of any time, including minutes, hours, days, weeks, months, or years. In certain embodiments, the sequential administration refers to administration separated by a time interval of one of at least 2 minutes, 5 minutes, 10 minutes, 30 minutes, 1 hour, 6 hours, 8 hours, 12 hours, 24 hours, 36 hours, 48 hours, 3 days, 4 days, 5 days, 6 days, 1 week, 2 weeks, 3 weeks, 1 month, 6 weeks, 2 months, 3 months, 4 months, 5 months, or 6 months.

In another aspect, the present application provides use of the CD80 protein variant and the fusion protein thereof in the preparation of a medicament for preventing or treating a disease or disorder. In another aspect, the present application provides the CD80 protein variant and the fusion protein thereof for preparing for preventing or treating a disease or disorder.

In another aspect, the present application provides a method for preventing or treating a tumor, which comprises administering to a subject in need thereof the CD80 protein variant and the fusion protein thereof described herein. In the present application,. Colorectal cancer may also be included.

In the present application, the tumor may include a solid tumor. In the present application, the tumor may include a non-solid tumor. In the present application, the tumor may include one or more selected from: malignant melanoma, breast cancer, gastric cancer, kidney cancer, non-small cell lung cancer, colon cancer, rectal cancer, head and neck squamous cell carcinoma, liver cancer, kidney cancer, mesothelioma bladder cancer, pancreatic cancer, ovarian cancer, endometrial cancer, and lymphoma. Without being bound by any theory, the following examples are intended only to illustrate the variant, the fusion protein, the preparation method, the use, etc., of the present application, and are not intended to limit the scope of the present application.

### Example

### Example 1. Preparation of CD80 Fusion Proteins

The CD80 fusion protein was obtained by linking a CD80 extracellular domain or a variant thereof to the N-terminus of an Fc fragment (amino acid sequence number: SEQ ID NO: 25) of human IgG1, wherein the CD80 extracellular domain or the variant thereof includes: wild-type CD80 ECD (SEQ ID NO: 1, extracellular domain of wild-type CD80), M12 (SEQ ID NO: 6), M13 (SEQ ID NO: 7), M14 (SEQ ID NO: 8), M15 (SEQ ID NO: 9), M16 (SEQ ID NO: 10), M18 (SEQ ID NO: 11), M19 (SEQ ID NO: 12), M20 (SEQ ID NO: 13), M21 (SEQ ID NO: 14), M22 (SEQ ID NO: 15), M23 (SEQ ID NO: 16), M24 (SEQ ID NO: 17), 004D25 (SEQ ID NO: 18), M26 (SEQ ID NO: 19), M27 (SEQ ID NO: 20), M28 (SEQ ID NO: 21), M29 (SEQ ID NO: 22), M30 (SEQ ID NO: 23), and M31 (SEQ ID NO: 24). An IgV domain of the CD80 protein, or a variant thereof includes: wild-type CD80 IgV (SEQ ID NO: 2), M09 (SEQ ID NO: 3), M10 (SEQ ID NO: 4), and M11 (SEQ ID NO: 5).

The CD80 extracellular domains or the variants thereof described above were separately linked to the N-terminus of the Fc fragment (amino acid sequence number: SEQ ID NO: 25) of human IgG1 to obtain CD80 fusion proteins, which were named: CD80-WT, CD80M12, CD80M13, CD80M14, CD80M15, CD80M16, CD80M18, CD80M19, CD80M20, CD80M21, CD80M22, CD80M23, CD80M24, CD80M25, CD80M26, CD80M27, CD80M28, CD80M29, CD80M30, CD80M31, CD80-IgV, CD80M09, CD80M10, and CD80M11.

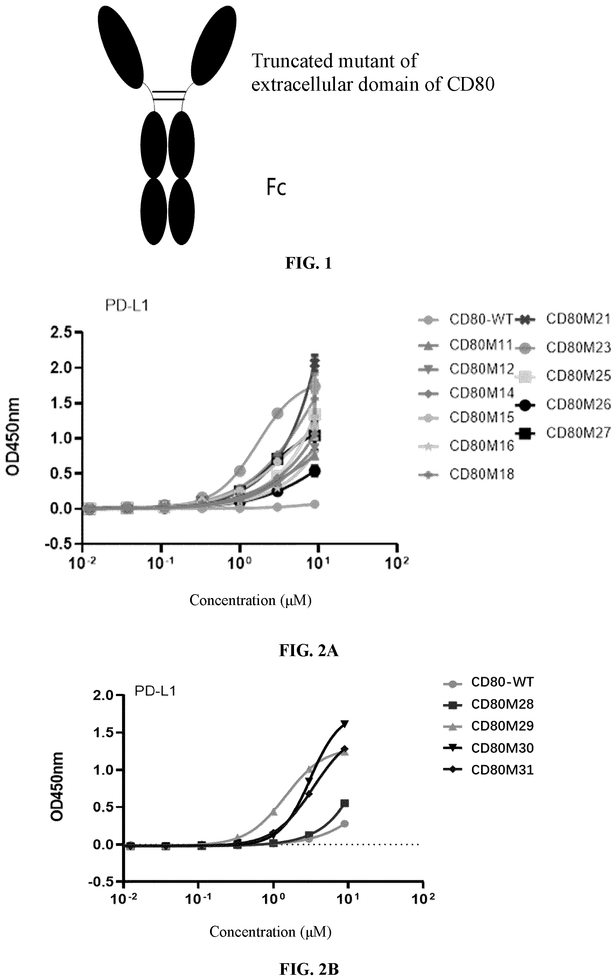

The specific structure of the CD80 fusion proteins is shown in FIG. 1.

Among them, CD80M12, CD80M11, CD80M14, CD80M15, CD80M16, CD80M26, CD80M27, CD80M28, CD80M29, CD80M30, and CD80M31 were stably expressed in moderate expression amounts, had good binding activity for PD-L1, CTLA-4, and/or CD28, and were capable of maintaining the affinity for PD-L1, CTLA-4, and/or CD28.

### Example 2. Assay of Activity of CD80 Fusion Proteins for Binding to Human PD-L1, CTLA-4, and CD28 Proteins

### (1) Evaluation of binding activity of the CD80 fusion proteins for PD-L1 by ELISA with CD80-WT as a control

An ELISA strip plate was coated with PD-L1 (human PD-L1/B7-H1/CD274 protein (His tag), Sino Biological) and incubated at 4 °C overnight. After the plate was washed with PBST, 10% fetal bovine serum was added, and the plate was blocked at 37 °C for 1 h. The CD80 fusion proteins at different concentrations were added, and the mixture was reacted at 37 °C for 1 h. After the plate was washed with PBST, a horseradish peroxidase-labeled goat anti-human IgG Fc secondary antibody (Goat anti-human IgG Fc antibody, horseradish peroxidase (HRP) conjugate, affinity purified, Invitrogen) was added, and the mixture was reacted at 37 °C for 30 min. After the plate was washed with PBST 5 times, 100 µL of TMB (eBioscience) was added to each well, and the plate was left to stand at room temperature (20 ± 5 °C) for 1-2 min in the dark. Then, 100 µL of 2 N H₂SO₄ stop buffer was added to each well to stop the substrate reaction. The OD values were read at 450 nm using a microplate reader, and the binding ability of the CD80 fusion proteins to PD-L1 was analyzed.

The results are shown in FIGs. 2A-2B. The results showed that under high concentration conditions, the CD80 protein variants described herein had significantly stronger binding activity for PD-L1 than CD80-WT.

### (2) Evaluation of binding activity of the CD80 fusion proteins for CTLA-4 by ELISA with CD80-WT as a control

An ELISA strip plate was coated with CTLA-4 (human CTLA-4/CD152 protein, ACROBiosystems) and incubated at 4 °C overnight. After the plate was washed with PBST, 10% fetal bovine serum was added, and the plate was blocked at 37 °C for 1 h. The CD80 fusion proteins at different concentrations were added, and the mixture was reacted at 37 °C for 1 h. After the plate was washed with PBST, a horseradish peroxidase-labeled goat anti-human IgG Fc secondary antibody (Goat anti-human IgG Fc antibody, horseradish peroxidase (HRP) conjugate, affinity purified, Invitrogen) was added, and the mixture was reacted at 37 °C for 30 min. After the plate was washed with PBST 5 times, 100 µL of TMB (eBioscience) was added to each well, and the plate was left to stand at room temperature (20 ± 5 °C) for 1-2 min in the dark. Then, 100 µL of 2 N H₂SO₄ stop buffer was added to each well to stop the substrate reaction. The OD values were read at 450 nm using a microplate reader, and the binding ability of the CD80 fusion proteins to CTLA-4 was analyzed.

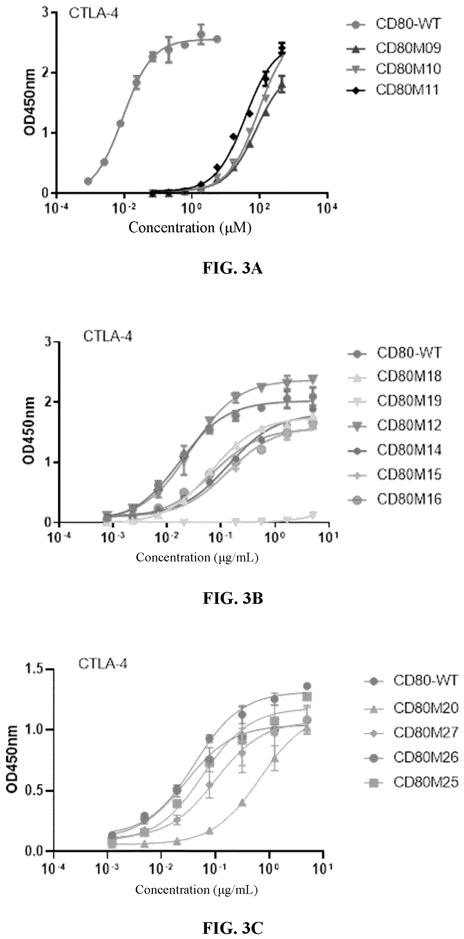

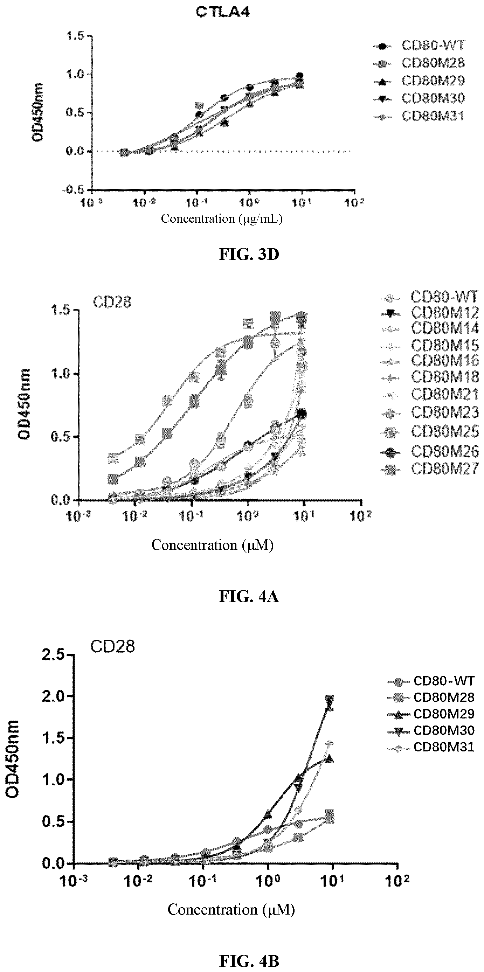

The results are shown in FIGs. 3A-3C. The results in FIG. 3A showed that except for CD80M09, CD80M10, CD80M11, CD80M19, and CD80M20, which had significantly weaker binding activity as compared to wild-type CD80-WT, the remaining variants had comparable binding activity for CTLA-4 to CD80WT.

### (3) Evaluation of binding activity of the CD80 fusion proteins for CD28 by ELISA with CD80-WT as a control

An ELISA strip plate was coated with CD28 (human CD28, Sino Biological) and incubated at 4 °C overnight. After the plate was washed with PBST, 10% fetal bovine serum was added, and the plate was blocked at 37 °C for 1 h. The CD80 fusion proteins at different concentrations were added, and the mixture was reacted at 37 °C for 1 h. After the plate was washed with PBST, a horseradish peroxidase-labeled goat anti-human IgG Fc secondary antibody (Goat anti-human IgG Fc antibody, horseradish peroxidase (HRP) conjugate, affinity purified, Invitrogen) was added, and the mixture was reacted at 37 °C for 30 min. After the plate was washed with PBST 5 times, 100 µL of TMB (eBioscience) was added to each well, and the plate was left to stand at room temperature (20 ± 5 °C) for 1-2 min in the dark. Then, 100 µL of 2 N H₂SO₄ stop buffer was added to each well to stop the substrate reaction. The OD values were read at 450 nm using a microplate reader, and the binding ability of the CD80 fusion proteins to CD28 was analyzed.

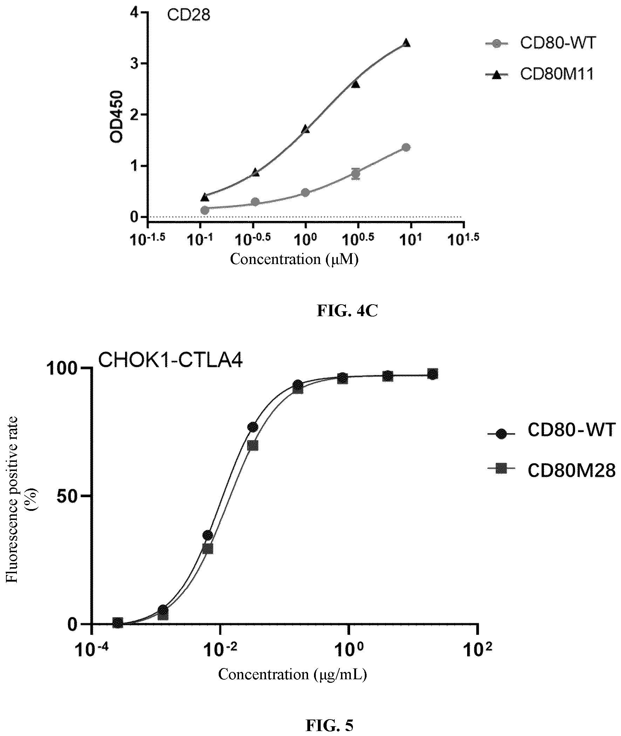

The results are shown in FIGs. 4A-4C. The results showed that CD80M11, CD80M23, CD80M25, CD80M27, CD80M29, CD80M30, and CD80M31 had significantly stronger binding activity for CD28 than CD80-WT, and the remaining fusion proteins had similar binding activity to CD80-WT.

### Example 3. Assay of Activity of CD80 Fusion Proteins for Binding to Human PD-L1, CTLA-4, and CD28 Positive Cells

### (1) Evaluation of binding of the CD80 fusion protein to CTLA-4 positive cells by FACS with CD80-WT as a control

CHOK1 cells over-expressing human CTLA-4 (CHOK1-CTLA-4) were collected and added to 1.5 mL EP tubes at 5 × 10⁵ cells per tube. The CD80 fusion proteins at different concentrations were added, and the tubes were incubated on ice for 30 min in the dark. After the tubes were washed with a FACS washing buffer, a PE fluorescence-labeled Goat anti-human IgG Fc secondary antibody (Invitrogen) was added, and the tubes were incubated on ice for 30 min in the dark. After the tubes were washed with a FACS washing buffer 2 times, 400 µL of 1% paraformaldehyde fixative (Solarbio) was added to each tube to fix the cells. The mixture was mixed uniformly and then determined on a platform for the PE fluorescence positive rate, and the binding ability of the CD80 fusion protein to CTLA-4 positive cells was analyzed.

The results are shown in FIG. 5. The results showed that CD80M28 had similar binding ability to cell surface CTLA-4 to CD80-WT.

### (2) Evaluation of binding of the CD80 fusion protein to PD-L1 positive cells by FACS with CD80-WT as a control

CHOK1 cells over-expressing human PD-L1 (CHOK1-PD-L1) were collected and added to 1.5 mL EP tubes at 5 × 10⁵ cells per tube. The CD80 fusion proteins at different concentrations were added, and the tubes were incubated on ice for 30 min in the dark. After the tubes were washed with a FACS washing buffer, a PE fluorescence-labeled Goat anti-human IgG Fc secondary antibody (Invitrogen) was added, and the tubes were incubated on ice for 30 min in the dark. After the tubes were washed with a FACS washing buffer 2 times, 400 µL of 1% paraformaldehyde fixative (Solarbio) was added to each tube to fix the cells. The mixture was mixed uniformly and then determined on a platform for the PE fluorescence positive rate, and the binding ability of the CD80 fusion protein to PD-L1 positive cells was analyzed.

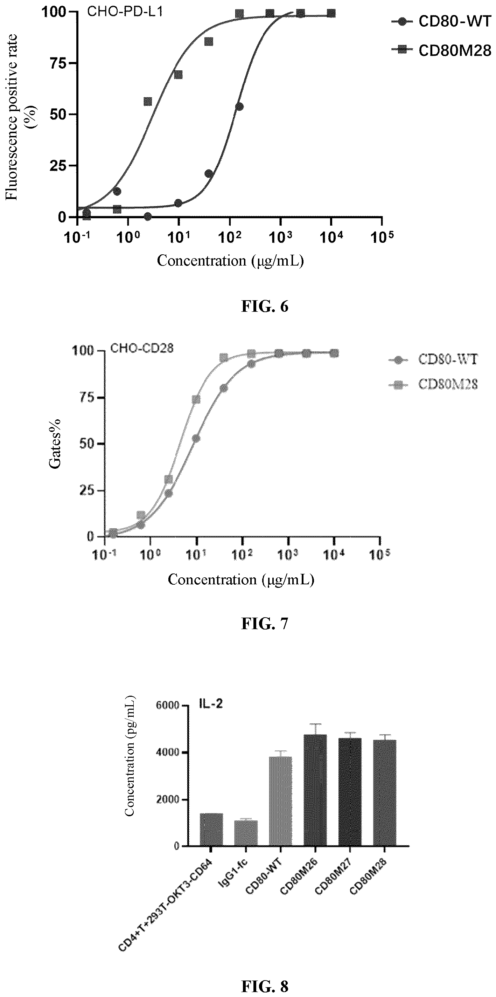

The results are shown in FIG. 6. The results showed that CD80M28 had stronger binding ability to cell surface PD-L1 than CD80-WT.

### (3) Evaluation of binding of the CD80 fusion protein to CD28 positive cells by FACS with CD80-WT as a control

CHOK1 cells over-expressing human CD28 (CHOK1-CD28) were collected and added to 1.5 mL EP tubes at 5 × 10⁵ cells per tube. The CD80 fusion proteins at different concentrations were added, and the tubes were incubated on ice for 30 min in the dark. After the tubes were washed with a FACS washing buffer, a PE fluorescence-labeled Goat anti-human IgG Fc secondary antibody (Invitrogen) was added, and the tubes were incubated on ice for 30 min in the dark. After the tubes were washed with a FACS washing buffer 2 times, 400 µL of 1% paraformaldehyde fixative (Solarbio) was added to each tube to fix the cells. The mixture was mixed uniformly and then determined on a platform for the PE fluorescence positive rate, and the binding ability of the CD80 fusion protein to CD28 positive cells was analyzed.

The results are shown in FIG. 7. The results showed that CD80M28 had a slightly stronger binding ability to cell surface CD28 than CD80-WT.

### Example 4. Assay of Activity of CD80 Fusion Proteins for Activating T Lymphocytes by Culture of aAPC/T Mixed Cells

The activity of the CD80 fusion proteins for activating T lymphocytes was evaluated using an experiment system of mixed culture of aAPC cells and CD4+ T lymphocytes, with CD80 fusion proteins CD80-Fc and IgG1-Fc as controls, 293T cells over-expressing OKT3 scFv and CD64 (293T-OKT3-CD64) as antigen-presenting cells (artificial APC, aAPC), and CD4+ T lymphocytes as effector cells.

293T-OKT3-CD64 cells were collected and added to a 96-well cell culture plate at 2.5 × 10⁴ cells per well. PBMC cells (MT-BIO) from healthy donors were isolated using a CD4+ T cell isolation kit (CD4+ T Cell Isolation Kit, human, Miltenyi Biotec) to obtain CD4+ T lymphocytes, which were added to the 96-well cell culture plate at 5 × 10⁴ cells per well. Then, 1200 nM CD80 fusion protein, IgG1-Fc protein (human IgG1 Fc protein, Tag Free, ACROBiosystems) and CD80 fusion protein CD80-Fc (human B7-1/CD80 protein, Fc Tag, ACROBiosystems) were added, the plate was incubated in an incubator at 37 °C for 24 h, and then supernatants were collected. IL-2 levels in the cell supernatants were determined using an IL-2 ELISA assay kit (HUMAN IL-2 ELISA KIT, ExCell Bio), and the activity of the CD80 fusion proteins in the culture system of aAPC/T mixed cells for activating T lymphocytes was analyzed.

The results are shown in FIG. 8. The results showed that CD80-WT, CD80M26, CD80M27, and CD80M28 were capable of activating lymphocytes in APC/T mixed cells, with activation activity similar to that of the CD80 molecule.

### Example 5. Evaluation of Activation Activity of Fusion Proteins for CD28 Signaling Pathway by Reporter Gene Assay

FCGR2B/TCR Activator/CHO (referred to as FCGR2B/CHO) cells were collected and added to a white clear-bottom 96-well plate (96 well white/clear bottom plate, Thermo) at 1.5 × 10⁴ cells/well. The fusion proteins at different concentrations were added to the cells. CD28 efficiency reporter (referred to as CD28 effector) effector cells were added at 2.5 × 10⁴ cells/well, and the 96-well plate was placed in an incubator at 37 °C with 5% CO₂ for culture for 5.5 h. 120 µL of luciferase assay reagent (Bright-Lumi^{™} firefly luciferase reporter gene assay kit, Beyotime) was added to each well, and the plate was incubated at room temperature for 10 min in the dark. The relative fluorescence intensity value of chemiluminescence was determined by using a multimode microplate reader, and the CD28 activation activity mediated by the fusion proteins in an FCGR2B/CHO cell + CD28 effector cell system.

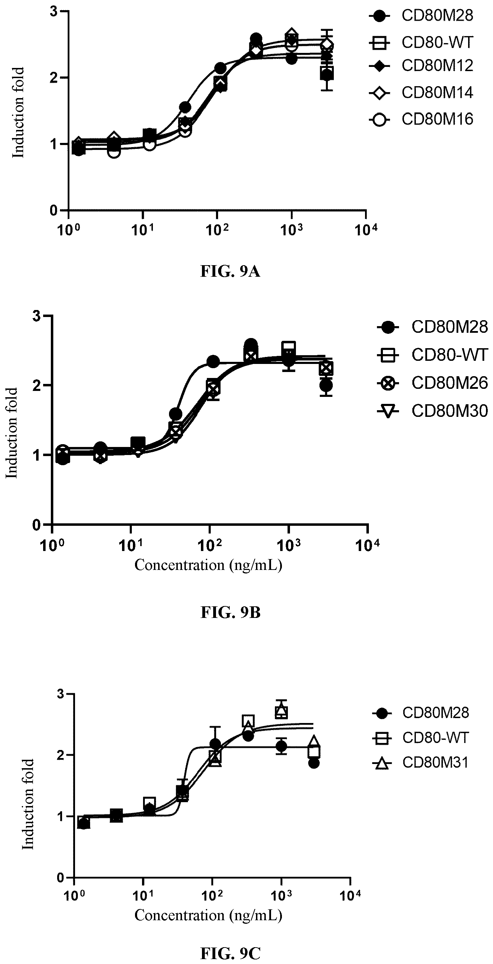

The results are shown in FIGs. 9A-9C. FIGs. 9A-9C showed that the fusion proteins all had CD28 activation activity, among which CD80M28 had stronger activity than CD80-WT, and CD80M12, CD80M14, CD80M16, CD80M26, CD80M30, and CD80M31 had activity close to CD80-WT.

### Example 6. Evaluation of Anti-Tumor Activity of CD80 Fusion Proteins Using CT26 Subcutaneous Xenograft Model

5 × 10⁵ CT26 cells resuspended in PBS (0.1 mL/mouse) were inoculated subcutaneously into the right dorsal side of experimental mice. The tumor growth was observed periodically. When the tumors grew to the mean volume of about 76 mm³, the mice were randomly grouped for administration according to the tumor size and the mouse body weight. The mice were divided into 5 groups of 6 mice each. G1 group was CD80M28, 2 mg/kg, G2 group was CD80M28, 0.5 mg/kg, G3 group was CD80M28, 0.1 mg/kg, G4 group was CD80-WTG1, 0.5 mg/kg, and G5 group was a vehicle control group, all of which were subjected to intraperitoneal administration 3 times a week for 3 doses in total.

On Day 22, there were mice in the vehicle control group that were euthanized due to tumor volume exceeding 3000 mm³, so the data on Day 22 were used as the basis for analysis. The mean tumor volume of the vehicle control group (G5) on Day 22 was 2189.79 mm³, and the mean tumor volume of the CD80M28 2 mg/kg TIW administration group on Day 22 was 865.57 mm³, with a relative tumor growth inhibition (TGI) rate of 60.47%, which was significantly different (p < 0.05) as compared to that of the control group. The mean tumor volume of the CD80M28 0.5 mg/kg TIW administration group on Day 22 was 877.01 mm³, with a relative tumor growth inhibition (TGI) rate of 59.95%, which was significantly different (p < 0.05) as compared to that of the control group. The mean tumor volume of the CD80M28 0.1 mg/kg TIW administration group on Day 22 was 1507.25 mm³, with a relative tumor growth inhibition (TGI) rate of 31.17%, which was not significantly different as compared to that of the control group. The mean tumor volume of the CD80-WTG1 0.5 mg/kg TIW administration group on Day 22 was 1337.30 mm³, with a relative tumor growth inhibition (TGI) rate of 38.93%, which was not significantly different as compared to that of the control group. The mice of the groups had normal body weight and no abnormal performance, and were in good general state.

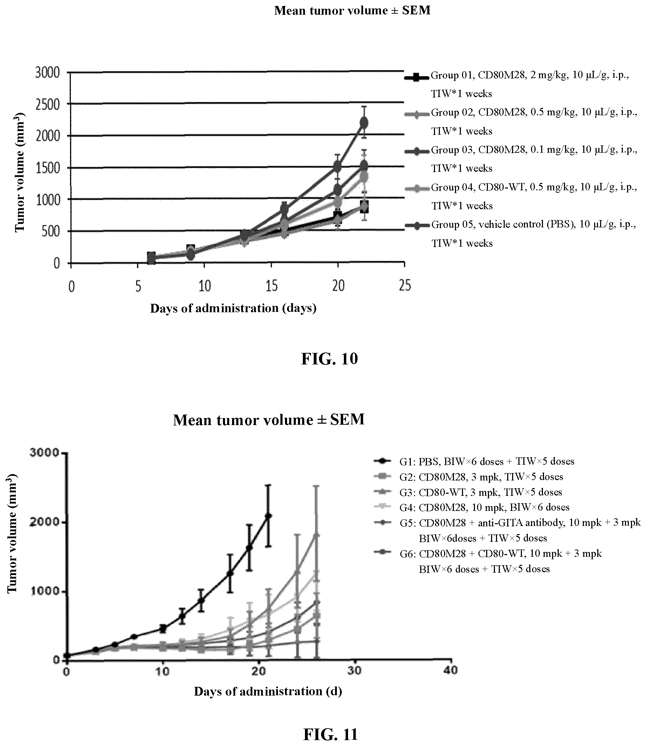

The results are shown in FIG. 10. The results showed that the inhibition of tumor growth by CD80M28 was dose-dependent, and the activity was significantly better than that of wild-type CD80-WT at the same dose.

### Example 7. Evaluation of Anti-Tumor Effect of Combination of CD80 Fusion Proteins and Treg Inhibitor Using CT26 Subcutaneous Xenograft Model

Taking an anti-GITR antibody as an example, the synergistic anti-tumor effect of the CD80 fusion proteins and Treg inhibitor was explored.

The synergistic effect of the combination of the anti-GITR antibody disclosed in WO2017096189A1 and the fusion protein of the present application was studied. Mouse colorectal cancer cells CT26 were inoculated subcutaneously into the right anterior flank of female PD1/GITR double humanized mice. When the tumors grew to about 80 mm³, the mice were grouped for administration. The mice were divided into 6 groups of 5 mice each, which were G1: a vehicle group, G2: a CD80M28 (3 mg/kg) group, G3: a CD80-WT (3 mg/kg) group, G4: an anti-GITR antibody (10 mg/kg) group, G5: a CD80M28 + anti-GITR antibody (3 + 10 mg/kg) group, and G6: a CD80-WT + anti-GITR antibody (3 + 10 mg/kg) group, respectively. CD80-WT and CD80M28 were administered intravenously three times a week, and the anti-GITR antibody was administered intraperitoneally twice a week for two weeks. Tumor volume and body weight were measured weekly and the changes in body weight and tumor volume of the tumor-bearing mice were recorded as a function of time of administration. Tumor growth inhibition TGI_{TV} (%) was calculated and statistically analyzed.

The results are shown in FIG. 11. The results showed that the tumor growth inhibition rates of CD80M28, CD80-WT, the anti-GITR antibody, CD80M28 + anti-GITR antibody, and CD80-WT + anti-GITR antibody were 86.43%, 64.57%, 69.98%, 89.06%, and 80.80%, respectively, and the tumor volume of the treatment groups was significantly lower than that of the vehicle control group (p < 0.05), showing a significant anti-tumor effect, and suggesting that the tumor growth was effectively inhibited. Moreover, the tumor volume of the CD80M28 + anti-GITR antibody combination treatment group was the smallest among all treatment groups, and the tumor inhibition activity of the combination treatment group was better than that of the single-dose groups.

### Example 8. Evaluation of Anti-Tumor Effect of Combination of Fusion Proteins and Anti-PD-1 Antibody Using CT26 Subcutaneous Xenograft Model

The synergistic effect of the combination of the CD80 fusion proteins of the present application and the anti-m-PD-1 antibody was studied. The anti-m-PD-1 antibody used in the study was purchased from Bioxcell, with the catalog number of BP0146.

Mouse colorectal cancer cells CT26 were inoculated subcutaneously into the right anterior flank of female Balb/c mice. The mean tumor volume was about 70.9 mm³ on Day 6 after inoculation, and the tumor-bearing mice were divided into 4 groups of 6 mice each by a randomized block method, including a Group 1 PBS group (hereinafter referred to as Group 1), a Group 2 CD80M28 (0.4 mg/kg) group (hereinafter referred to as Group 2), a Group 3 anti-mPD-1 antibody (5 mg/kg) group (hereinafter referred to as Group 3), and a Group 4 anti-mPD-1 antibody (5 mg/kg) + CD80M28 (0.4 mg/kg) group (hereinafter referred to as Group 4). The administration volume of the groups was 10 mL/kg. Group 1 and Group 2 were subjected to administration via tail vein 3 times a week for 1 consecutive week; Group 3 was subjected to intraperitoneal administration 2 times a week for 2 consecutive weeks for 4 doses in total; Group 4 was subjected to combination treatment: the anti-mPD-1 antibody (5 mg/kg) was administered intraperitoneally 2 times a week for 2 consecutive weeks, and CD80M28 (0.4 mg/kg) was administered 3 times a week for 1 consecutive week for 7 doses in total.

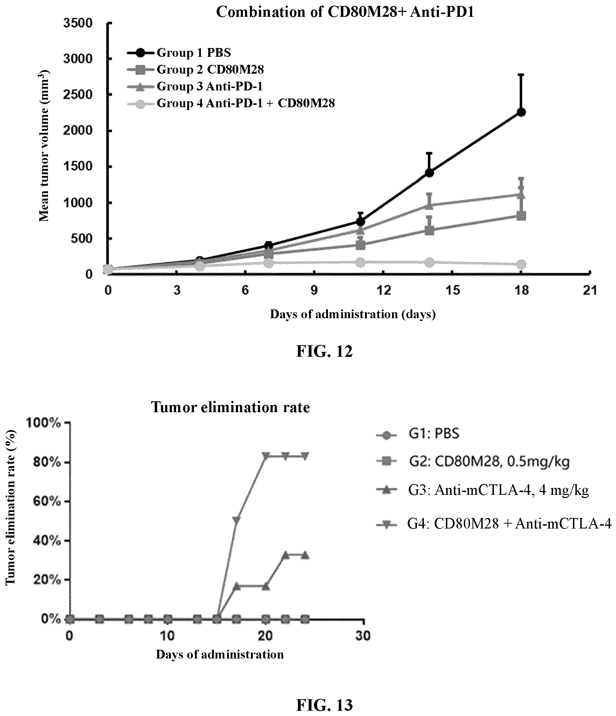

The results are shown in FIG. 12. The results showed that the tumor volume of the Group 1 group was 2264.23 ± 514.76 mm³ on Day 18 after the start of the administration. The tumor volume of the Group 3 group was 1109.87 ± 224.72 mm³, with a tumor growth inhibition (TGI) rate of 52.64%, which was not statistically different (P > 0.05) as compared to the control group; the tumor volume of the Group 2 group was 821.79 ± 387.96 mm³, with a tumor growth inhibition (TGI) rate of 65.76%, which was statistically different (P < 0.05) as compared to the control group; the tumor volume of the Group 4 group was 140.49 ± 34.58 mm³, with a tumor growth inhibition (TGI) rate of 96.83%, which was statistically different (P < 0.01) as compared to the control group.

The anti-tumor activity of the combination treatment group was significantly better than that of the single-dose groups, and the combination treatment group had a significant synergistic effect.

### Example 9. Evaluation of Anti-Tumor Effect of Combination of Fusion Proteins and Anti-CTLA-4 Antibody Using MC38 Subcutaneous Xenograft Model

The synergistic effect of the combination of the CD80 fusion proteins of the present application and the anti-m-CTLA-4 antibody was studied. The anti-m-CTLA-4 antibody was purchased from Bioxcell, with the catalog number of BP0164. Mouse colorectal cancer cells MC38 were inoculated subcutaneously into the right anterior flank of female C57BL/6 mice. The mean tumor volume was about 78 mm³ on Day 7 after inoculation, and the tumor-bearing mice were divided into 4 groups of 6 mice each by a random block method, including a Group 1 PBS group (hereinafter referred to as Group 1), a Group 2 CD80M28 (0.5 mg/kg) group (hereinafter referred to as Group 2), a Group 3 CTLA-4 (4 mg/kg) group (hereinafter referred to as Group 3), and a Group 4 CTLA-4 (5 mg/kg) + CD80M28 (0.4 mg/kg) group (hereinafter referred to as Group 4). CD80M28 was administered intravenously once and the CTLA-4 antibody was administered intraperitoneally twice a week for a total of three doses.

On Day 17 of the experiment, there were animals showing disappearance of subcutaneous tumors in both the anti-mCTLA-4 4 mg/kg (G3) group and the CD80M28 + anti-mCTLA-4 (G4) combination treatment group. By Day 24 of the experiment, there were two animals showing disappearance of subcutaneous tumors in the anti-mCTLA-4 4 mg/kg (G3) group, with a tumor elimination rate of 33.3%; there were five animals showing disappearance of subcutaneous tumors in the CD80M28 + anti-mCTLA-4 (G4) combination treatment group, with a tumor elimination rate of 83.3 %, resulting in better activity than the single-dose groups. The results are shown in FIG. 13.

### Example 10. Cytokine Release Induced by CD80 Fusion Protein

The ability of the CD80 fusion protein to induce cytokine release from PBMC was evaluated by ELISA with the CD3 antibody hOKT3 and the CD28 antibody TGN1412 as controls.

The CD80 fusion proteins at different concentrations, the CD3 antibody hOKT3 (human CD3 (humanized OKT3) antibody-human IgG4 (GMP-grade), Sino Biological), the CD28 antibody TGN1412 (self-produced), and the IgG1-Fc protein (human IgG1 Fc protein, Tag Free, ACROBiosystems) were added to a 96-well cell culture plate and air dried at room temperature overnight. After the plate was washed with normal saline 2 times, 1 × 10⁵ PBMC cells from healthy donors (Schbio Biotech) were added to each well, the plate was incubated in an incubator at 37 °C for 24 h, and then supernatants were collected. TNF-α and IL-6 levels in the supernatants were determined using a TNF-α ELISA assay kit (HUMAN TNF-α ELISA KIT, ExCell Bio) and an IL-6 ELISA assay kit (Human IL-6 Precoated ELISA Kit, DAKEWE), respectively, and the ability of the CD80 fusion protein to induce cytokine release from PBMC was analyzed.

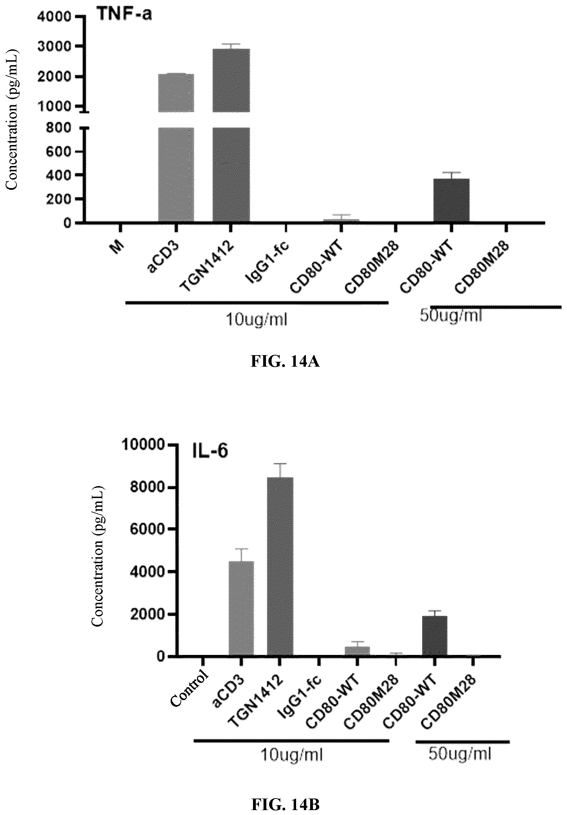

The results are shown in FIGs. 14A-14B. The results showed that CD80-WT and CD80M28 did not stimulate cytokine release and therefore had good safety.

### Example 11. Charge Heterogeneity Analysis of CD80 Fusion Proteins

Taking CD80M26 and CD80M28 as examples, charge heterogeneity analysis of the CD80 variant fusion proteins was performed. Capillary tubes were rinsed at room temperature, and a mixed standard sample solution and a detection sample solution were prepared. The CD80M28 solution was prepared with the following components: 50 µL of 1% MC, 50 µL of 8.6 mM urea, 10 µL of 200 mM IDA, 7 µL of 500 mM arginine, 25 µL of 1 mg/mL CD80M28, 1.6 µL of ampholytes 3-10, and 6.4 µL of ampholytes 5-8. The CD80-WT or CD80M26 solution was prepared with the following components: 50 µL of 1% MC, 50 µL of 8.6 mM urea, 10 µL of 200 mM IDA, 7 µL of 500 mM arginine, 25 µL of 1mg/mL CD80-WT or CD80M26, 1.2 µL of ampholytes 3-10, and 10.8 µL of ampholytes 5-8. Electrophoresis was performed with the injection pressure selected to be 2 bar and the injection time set to be 100 s, and chromatograms for the compounds separated by differences in charge heterogeneity were collected. The acidic and basic variants were quantitatively analyzed by an area normalization method.

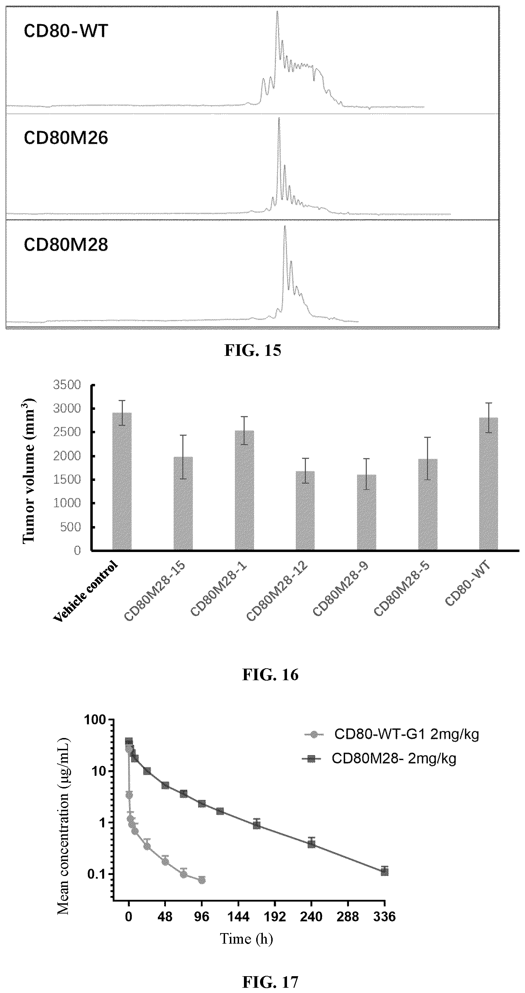

The results are shown in FIG. 15. The results showed that the charge heterogeneity of CD80M26 and CD80M28 was significantly less than that of CD80-WT. Proteins with charge heterogeneity had more homogeneous quality control and better druggability.

### Example 12. Analysis of Sialic Acid Proportion of CD80M28 Samples from Different Cell Clones by HPLC-FLD Assay

Taking CD80M28 as an example, the effect of sialic acid proportion was assayed. The sialic acid content was determined by an HPLC-FLD assay. The protein test samples were each buffer-exchanged into PBS 5 times and then determined for the protein concentration. Then the protein was diluted to 1 mg/mL, 4 M acetic acid was added to the protein sample according to a ratio of 1:1, and the mixture was mixed uniformly and incubated at 80 °C for 2 h. 50 µL of DMB solution was added to the above system, and the mixture was incubated at 50 °C for 2.5 h. Two common sialic acid Neu5Ac (NA)/Neu5Gc (NG) quantitative standards commonly found in glycoproteins were prepared into different concentrations, which were used for plotting standard curves, and quantitative analysis was performed on Neu5Ac (NA)/Neu5Gc (NG).

| Sample name | Neu5Ac content in glycoproteins by MR (Mol/Mol) |
|---|---|
| CD80M28-15 | 14.78 |
| CD80M28-12 | 11.98 |
| CD80M28-1 | 0.76 |
| CD80M28-9 | 8.82 |
| CD80M28-5 | 4.98 |

### Example 13. Evaluation of Anti-Tumor Effect of CD80M28 with Different Degrees of Sialic Acid Modification Using CT26 Subcutaneous Xenograft Model

Mouse colorectal cancer cells CT26 were inoculated subcutaneously into the right anterior flank of 20 female BABL/C mice. When the tumors grow to about 80 mm³, the mice were divided into 7 groups of 6 mice each, including a G1 group vehicle control (PBS), a G2 group CD80M28-15, a G3 group CD80M28-1, a G4 group CD80M28-12, a G5 group CD80M28-99, a G6 group CD80M28-5, and a G7 group CD80-WT. The administration was performed once every three days for 3 doses in total.

The mean tumor volume of the vehicle control group at the end of the experiment was 2906.96 mm³, the mean tumor volume of the CD80M28-15 treatment group at the end of the experiment was 1979.69 mm³, with a relative tumor growth inhibition (TGI) rate of 31.90%; the mean tumor volume of the CD80M28-1 treatment group at the end of the experiment was 2536.42 mm³, with a relative tumor growth inhibition (TGI) rate of 12.75%; the mean tumor volume of the CD80M28-12 treatment group at the end of the experiment was 1683.28 mm³, with a relative tumor growth inhibition (TGI) rate of 42.09%; the mean tumor volume of the CD80M28-9 treatment group at the end of the experiment was 1612.37 mm³, with a relative tumor growth inhibition (TGI) rate of 44.53%; the mean tumor volume of the CD80M28-5 treatment group at the end of the experiment was 1940.70 mm³, with a relative tumor inhibition (TGI) rate of 33.24%; the mean tumor volume of the CD80-WT treatment group at the end of the experiment was 2805.13 mm³, with a relative tumor inhibition (TGI) rate of 3.50%.

The results are shown in FIG. 16. The results showed that the tumor inhibitory effect of CD80M28 with different sialic acid contents was better than that of wild-type CD80-WT, and when the sialic acid content was greater than 0.76, the tumor growth inhibition rates of the CD80 fusion proteins were more significant. Particularly, when the sialic acid content was more than 1, the tumor growth inhibition rates of the CD80 fusion proteins were significantly increased. This suggests that the improvement of sialic acid content is helpful in improving the tumor inhibitory effect of the CD80 fusion proteins.

### Example 14. Pharmacokinetic Animal Experiment

After single intravenous infusions of CD80-WT-G1 (CD80-WT) and CD80M28 stock solutions in Sprague Dawley rats, the pharmacokinetic characteristics were compared between two test substances by determining the concentrations of the test substances in serum to provide data support for subsequent experiments.

### Analysis method:

Quantitative range: 50.0-3200 ng/mL

**Experimental design: Table 1**

| Group | Test substance | Dose (mg/kg) | Administration concentration (mg/mL) | Unit administration volume (mL/kg) |
|---|---|---|---|---|
| A | CD80-WT-G1 | 2 | 0.4 | 5 |
| B | CD80M28 stock solution | 2 | 0.4 | 5 |

| | | | | |
|---|---|---|---|---|
| Note: the administration mode was intravenous infusion, and the infusion time was 4 minutes. | | | | |

### Sampling:

A total of 224 samples were taken at 14 time points, including 0 min before administration, 5 min, 30 min, 2 h, 4 h, 8 h, 24 h, 48 h, 72 h, 96 h, 120 h, 168 h, 240 h, and 336 h after administration.

**Experimental results: Table 2**

| Mean pharmacokinetic parameters of the groups after single intravenous infusions of CD80-WT-G1 (CD80-WT) and CD80M28 stock solutions in Sprague Dawley rats (Mean ± SD, n = 8) | | | | |
|---|---|---|---|---|
| Parameter | Unit | Mean ± SD | | *P* |
| | | CD80-WT-G1 | CD80M28 | |
| | | *iv* 2mg/kg | *iv 2*mg/kg | |
| Kₑₗ | 1/hr | 0.0198±0.00720 | 0.0166±0.00643 | 0.368 |
| t_{1/2} | hr | 41.5±21.4 | 46.3±14.2 | 0.605 |
| Tₘₐₓ | hr | 0.0833 | 0.0833 (0.0833 0.500) | 0.334 |
| Cₘₐₓ | µg/mL | 26.3±5.81 | 37.8±3.55 | 0.000 |
| AUC₍₀₋ₜ₎ | hr*µg/mL | 35.4±10.1 | 931±93.8 | 0.000 |
| AUC_{inf} | hr*µg/mL | 39.8±11.2 | 946±95.1 | 0.000 |
| AUCₑₓₜᵣ | % | 10.9±4.50 | 1.59±1.35 | 0.000 |
| V_{d} | mL/kg | 3030±1130 | 141±40.9 | 0.000 |
| CLₛ | mL/hr/kg | 54.1±16.7 | 2.14±0.236 | 0.000 |
| MRₗₐₛₜ | hr | 18.6±3.01 | 48.3±8.09 | 0.000 |

| | | | | |
|---|---|---|---|---|
| Note: the mean value of Tₘₐₓ was expressed by median or median and range (minimum, maximum); independent sample *t*-test analysis was performed using SPSS software, *P* > 0.05: there were no statistical differences between groups, and *P* < 0.05: there were statistical differences between groups. | | | | |

After single intravenous infusions of 2 mg/kg CD80-WT-G1 stock solution and 2 mg/kg CD80M28 stock solution in Sprague Dawley rats, the Cₘₐₓ, AUC₍₀₋₁₎, and AUC_{inf} of CD80M28 were 1.44, 26.3, and 23.8 times, respectively, the CL was 0.0396 times, and the MRT was 2.60 times, as compared to CD80- WT-G 1. The results of the t-test analysis performed using SPSS software showed that except for Kₑₗ, t_{1/2}, and Tₘₐₓ, which were not statistically different, there were statistical differences in the remaining parameters Cₘₐₓ, AUC₍₀₋₁₎, AUC_{inf}, AUCₑₓₜᵣ, V_{d}, CL, and MRT between the CD80-WT-G1 and CD80M28 dose groups. The mean plasma concentration-time curves of the groups after single intravenous infusions of CD80-WT-G1 and CD80M28 stock solutions in Sprague Dawley rats are shown in FIG. 17. The results suggested that CD80M28 was exposed more, was cleared more slowly, and remained exposed for a longer period of time in rats.

## Claims

1. A CD80 protein variant, comprising a functionally active fragment of a CD80 protein, or a variant thereof, wherein the variant has mutations at one or more amino acid sites as compared to a corresponding functionally active fragment of wild-type CD80, and the variant has one or more of the following properties:
a) binding to PD-L1;
b) binding to CTLA-4;
c) binding to CD28;
d) activating the activity of T lymphocytes; and
e) inhibiting the growth and/or proliferation of a tumor or tumor cell.

2. The CD80 protein variant according to claim 1, wherein the PD-L1 is human PD-L1, the CTLA-4 is human CTLA-4, and the CD28 is human CD28.

3. The CD80 protein variant according to claim 1 or 2, wherein the functionally active fragment of the CD80 protein comprises an extracellular domain (ECD) of the CD80 protein, or a fragment thereof.

4. The CD80 protein variant according to claim 3, wherein the fragment of the extracellular domain of the CD80 protein comprises an IgV domain of the CD80 protein.

5. The CD80 protein variant according to any one of claims 1-4, comprising, as compared to a corresponding extracellular domain of the wild-type CD80 protein, or a fragment thereof, amino acid mutations at one or more amino acid residues selected from: N55, N64, N152, N173, N177, N192, and N198.

6. The CD80 protein variant according to any one of claims 1-5, comprising mutations at any one of groups of amino acid positions selected from:
1) N64;
2) N55 and N64;
3) N55, N64, and N152;
4) N55, N64, and N192;
5) N55, N64, and N198;
6) N55, N64, N152, and N198;
7) N55, N64, N152, and N192;
8) N55, N64, N152, N173, N177, and N198;
9) N55, N64, N152, N173, and N198; and
10) N55, N64, N152, N177, and N198.

7. The CD80 protein variant according to any one of claims 1-6, comprising one or more amino acid mutations selected from: N55A/Q, N64A, N152A, N173Q, N177A, N192A, and N198A/Q.

8. The CD80 protein variant according to any one of claims 1-7, comprising any one of groups of amino acid mutations selected from:
1) N64A;
2) N55A and N64A;
3) N55A, N64A, and N152A;
4) N55A, N64A, and N192A;
5) N55A, N64A, and N198A;
6) N55A, N64A, N152A, and N198A;
7) N55A, N64A, N152A, and N192A;
8) N55Q, N64A, N152A, and N198Q;
9) N55Q, N64A, N152A, N173Q, N177A, and N198Q;
10) N55Q, N64A, N152A, N173Q, and N198Q; and
11) N55Q, N64A, N152A, N177A, and N198Q.

9. The CD80 protein variant according to any one of claims 1-8, comprising at least one first-class amino acid mutation capable of maintaining or enhancing the stability of the CD80 protein variant.

10. The CD80 protein variant according to claim 9, comprising first-class amino acid mutations at one or more of the following amino acid positions: N55, N64, N152, N192, and N198.

11. The CD80 protein variant according to any one of claims 9-10, comprising first-class amino acid mutations at any one of groups of amino acid positions selected from:
1) N64;
2) N55 and N64;
3) N55, N64, and N152;
4) N55, N64, and N192;
5) N55, N64, and N198;
6) N55, N64, N152, and N198; and
7) N55, N64, N152, and N192.

12. The CD80 protein variant according to any one of claims 9-11, comprising one or more of the following first-class amino acid mutations: N55A/Q, N64A, N152A, N192A, and N198A/Q.

13. The protein variant according to any one of claims 9-12, comprising any one of groups of first-class amino acid mutations selected from:
1) N64A;
2) N55A and N64A;
3) N55A, N64A, and N152A;
4) N55A, N64A, and N192A;
5) N55A, N64A, and N198A;
6) N55A, N64A, N152A, and N198A;
7) N55A, N64A, N152A, and N192A; and
8) N55Q, N64A, N152A, and N198Q.

14. The CD80 protein variant according to any one of claims 1-13, comprising at least one second-class amino acid mutation capable of retaining the binding activity of the CD80 protein variant.

15. The CD80 protein variant according to claim 14, comprising second-class amino acid mutations at one or more of the following amino acid positions: N152; and N192 and N198.

16. The CD80 protein variant according to any one of claims 14-15, comprising one or more of the following second-class amino acid mutations: N152A; and N192A and N198A/Q.

17. The CD80 protein variant according to any one of claims 14-16, comprising second-class amino acid mutations at positions N55 and/or N64.

18. The CD80 protein variant according to any one of claims 14-17, comprising second-class amino acid mutations of N55A/Q and/or N64A.

19. The CD80 protein variant according to any one of claims 1-18, comprising at least one third-class amino acid mutation capable of retaining the affinity of the CD80 protein variant.

20. The CD80 protein variant according to claim 19, comprising third-class amino acid mutations at one or more of the following positions: N55, N64, N152, and N198.

21. The CD80 protein variant according to any one of claims 19-20, comprising one or more of the following third-class amino acid mutations: N55A/Q, N64A, N152A, and N198A.

22. The CD80 protein variant according to any one of claims 19-21, comprising a third-class amino acid mutation at position N192.

23. The CD80 protein variant according to any one of claims 19-22, comprising a third-class amino acid mutation of N192A.

24. The CD80 protein variant according to any one of claims 1-23, comprising at least one fourth-class amino acid mutation capable of enhancing the binding of the CD80 protein variant to CD28 and/or PD-L1.

25. The CD80 protein variant according to claim 24, comprising fourth-class amino acid mutations at positions N173 and/or N177.

26. The CD80 protein variant according to any one of claims 24-25, comprising fourth-class amino acid mutations of N173Q and/or N177A.

27. The CD80 protein variant according to any one of claims 1-26, wherein numbers of the amino acid positions are determined with reference to position numbers of amino acids in SEQ ID NO: 1.

28. The CD80 protein variant according to any one of claims 1-27, comprising an amino acid sequence set forth in any one of SEQ ID NO: 5, SEQ ID NO: 6, SEQ ID NO: 8, SEQ ID NO: 9, SEQ ID NO: 10, SEQ ID NO: 19, SEQ ID NO: 20, SEQ ID NO: 21, SEQ ID NO: 22, SEQ ID NO: 23, and SEQ ID NO: 24.

29. The CD80 protein variant according to any one of claims 1-28, having a sialic acid content of greater than or equal to about 0.7.

30. A fusion protein, comprising the CD80 protein variant according to any one of claims 1-29.

31. The fusion protein according to claim 30, further comprising an immunoglobulin Fc region or a variant thereof.

32. The fusion protein according to claim 31, wherein the CD80 protein variant and the immunoglobulin Fc region are linked directly or indirectly.

33. The fusion protein according to any one of claims 31-32, wherein the immunoglobulin Fc region comprises an Fc region of IgG.

34. The fusion protein according to claim 33, wherein the IgG is selected from: IgG1 and IgG4.

35. The fusion protein according to any one of claims 31-34, wherein the CD80 protein variant is located at the N-terminus or C-terminus of the immunoglobulin Fc region.

36. The fusion protein according to any one of claims 31-35, wherein the immunoglobulin Fc region comprises an amino acid sequence selected from: SEQ ID NO: 25 and SEQ ID NO: 26.

37. The fusion protein according to any one of claims 30-36, comprising an amino acid sequence set forth in any one of SEQ ID NOs: 27-37.

38. A polypeptide, comprising the CD80 protein variant according to any one of claims 1-29 or the fusion protein according to any one of claims 30-37.

39. One or more isolated nucleic acid molecules, encoding the CD80 protein variant according to any one of claims 1-29, the fusion protein according to any one of claims 30-37, or the polypeptide according to claim 38.

40. A vector, comprising the nucleic acid molecules according to claim 39.

41. A host cell, comprising the nucleic acid molecules according to claim 39 or the vector according to claim 40.

42. A method for preparing the CD80 protein variant according to any one of claims 1-29 or the fusion protein according to any one of claims 30-37, comprising culturing the cell according to claim 41 under conditions that enable the fusion protein to be expressed.

43. A pharmaceutical composition, comprising the CD80 protein variant according to any one of claims 1-29, the fusion protein according to any one of claims 30-37, the polypeptide according to claim 38, the nucleic acid molecules according to claim 39, the vector according to claim 40, and/or the host cell according to claim 41, and optionally a pharmaceutically acceptable carrier.

44. A pharmaceutical combination, comprising the CD80 protein variant according to any one of claims 1-19, the fusion protein according to any one of claims 30-37, or the polypeptide according to claim 38, and an immune checkpoint inhibitor.

45. The pharmaceutical combination according to claim 44, wherein the immune checkpoint comprises PD-1, PD-L1, GITR, and/or CTLA-4.

46. The pharmaceutical combination according to any one of claims 44-45, wherein the immune checkpoint inhibitor comprises one or more selected from: a PD-1 antibody, a PD-L1 antibody, a CTLA-4 antibody, and a GITR antibody.

47. A method for modulating an immune response in a subject, comprising administering the CD80 protein variant according to any one of claims 1-29, the fusion protein according to any one of claims 30-37, or the polypeptide according to claim 38.

48. The method according to claim 41, wherein the method for modulating an immune response in a subject comprises increasing an immune response.

49. The method according to any one of claims 47-48, wherein the CD80 protein variant exhibits increased binding affinity for PD-L1 as compared to a wild-type CD80 protein or a fragment thereof.

50. The method according to any one of claims 47-49, wherein the CD80 protein variant exhibits increased binding affinity for CD28 as compared to a wild-type CD80 protein or a fragment thereof.

51. The method according to any one of claims 47-50, wherein the CD80 protein variant exhibits enhanced activity for activating T lymphocytes as compared to a wild-type CD80 protein or a fragment thereof.

52. Use of the CD80 protein variant according to any one of claims 1-29, the fusion protein according to any one of claims 30-37, the polypeptide according to claim 38, the nucleic acid molecules according to claim 39, the vector according to claim 40, the host cell according to claim 41, the pharmaceutical composition according to claim 43, or the pharmaceutical combination according to any one of claims 44-46, in the preparation of a medicament for preventing and/or treating a disease and/or disorder.

53. The use according to claim 52, wherein the disease and/or disorder comprises a tumor.

54. The use according to claim 53, wherein the tumor comprises a solid tumor and/or a hematological tumor.

55. The use according to any one of claims 53-54, wherein the tumor comprises a tumor that is responsive to the proliferation of central memory T cells.

56. The use according to any one of claims 53-55, wherein the tumor is selected from one or more of: malignant melanoma, breast cancer, gastric cancer, kidney cancer, non-small cell lung cancer, colon cancer, rectal cancer, head and neck squamous cell carcinoma, liver cancer, kidney cancer, mesothelioma bladder cancer, pancreatic cancer, ovarian cancer, endometrial cancer, and lymphoma.

57. A method for preventing and/or treating a disease and/or disorder, comprising administering to a subject in need thereof the CD80 protein variant according to any one of claims 1-29, the fusion protein according to any one of claims 30-37, the polypeptide according to claim 38, the nucleic acid molecules according to claim 39, the vector according to claim 40, the host cell according to claim 41, the pharmaceutical composition according to claim 43, or the pharmaceutical combination according to any one of claims 44-46.

58. The method according to claim 57, wherein the disease and/or disorder comprises a tumor.

59. The method according to claim 58, wherein the tumor comprises a solid tumor and/or a hematological tumor.

60. The method according to any one of claims 58-59, wherein the tumor comprises a tumor that is responsive to the proliferation of central memory T cells.

61. The method according to any one of claims 58-60, wherein the tumor is selected from one or more of: malignant melanoma, breast cancer, gastric cancer, kidney cancer, non-small cell lung cancer, colon cancer, rectal cancer, head and neck squamous cell carcinoma, liver cancer, kidney cancer, mesothelioma bladder cancer, pancreatic cancer, ovarian cancer, endometrial cancer, and lymphoma.

62. The CD80 protein variant according to any one of claims 1-29, the fusion protein according to any one of claims 30-37, the polypeptide according to claim 38, the nucleic acid molecules according to claim 39, the vector according to claim 40, the host cell according to claim 41, the pharmaceutical composition according to claim 43, or the pharmaceutical combination according to any one of claims 44-46, for use in preventing and/or treating a disease and/or disorder.

63. The CD80 protein variant, the fusion protein, the nucleic acid molecules, the vector, the host cell, the pharmaceutical composition, or the pharmaceutical combination according to claim 62, wherein the disease and/or disorder comprises a tumor.

64. The CD80 protein variant, the fusion protein, the nucleic acid molecules, the vector, the host cell, the pharmaceutical composition, or the pharmaceutical combination according to claim 63, wherein the tumor comprises a solid tumor and/or a hematological tumor.

65. The CD80 protein variant, the fusion protein, the nucleic acid molecules, the vector, the host cell, the pharmaceutical composition, or the pharmaceutical combination according to any one of claims 63-64, wherein the tumor comprises a tumor that is responsive to the proliferation of central memory T cells.

66. The CD80 protein variant, the fusion protein, the nucleic acid molecules, the vector, the host cell, the pharmaceutical composition, or the pharmaceutical combination according to any one of claims 63-65, wherein the tumor is selected from one or more of: malignant melanoma, breast cancer, gastric cancer, kidney cancer, non-small cell lung cancer, colon cancer, rectal cancer, head and neck squamous cell carcinoma, liver cancer, kidney cancer, mesothelioma bladder cancer, pancreatic cancer, ovarian cancer, endometrial cancer, and lymphoma.

67. A method for detecting the frequency and/or proliferation of central memory T cells in a subject, comprising administering the CD80 protein variant according to any one of claims 1-29, the fusion protein according to any one of claims 30-37, or the polypeptide according to claim 38.

68. The method according to claim 67, wherein the central memory T cells are CD95+ and CD28+ cells.

69. The method according to claim 67, wherein the central memory T cells are CD4+ central memory T cells and/or CD8+ central memory T cells.

70. The CD80 protein variant according to any one of claims 1-29, the fusion protein according to any one of claims 30-37, the polypeptide according to claim 38, the nucleic acid molecules according to claim 39, the vector according to claim 40, the host cell according to claim 41, the pharmaceutical composition according to claim 43, or the pharmaceutical combination according to any one of claims 44-46, for use in enhancing the proliferation or frequency of central memory T cells.
